(19) Europäisches Patentamt
European Patent Office
Office européen des brevets

(11) EP 4 796 633 A1

(12) **EUROPEAN PATENT APPLICATION**
published in accordance with Art. 153(4) EPC

(43) Date of publication:
**26.08.2026 Bulletin 2026/35**

(21) Application number: **24890746.1**

(22) Date of filing: **14.11.2024**

(51) International Patent Classification (IPC):
***C12N 15/113*** *(2010.01)* ***A61K 31/713*** *(2006.01)*
***A61P 9/00*** *(2006.01)* ***A61K 48/00*** *(2006.01)*

(52) Cooperative Patent Classification (CPC):
**A61K 31/713; A61K 48/00; A61P 9/00; C12N 15/113**

(86) International application number:
**PCT/CN2024/131910**

(87) International publication number:
**WO 2025/103391 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**15.11.2023 PCT/CN2023/131684**
**07.12.2023 PCT/CN2023/137054**
**07.02.2024 PCT/CN2024/076513**
**22.03.2024 PCT/CN2024/083312**
**10.05.2024 PCT/CN2024/092119**
**15.05.2024 PCT/CN2024/093364**
**02.07.2024 PCT/CN2024/103107**
**19.08.2024 PCT/CN2024/113062**

(71) Applicant: **Jacoray Pharmaceutical Technology Co., Ltd.**
**Beijing 100176 (CN)**

(72) Inventors:
- **ZHENG, Qian**
  **Beijing 100176 (CN)**
- **WANG, Yanping**
  **Beijing 100176 (CN)**

(74) Representative: **Staeger & Sperling Partnerschaftsgesellschaft mbB**
**Sonnenstraße 19**
**80331 München (DE)**

Remarks:
The complete document including Reference Table(s) and the Sequence Listing(s) can be downloaded from the EPO website

(54) **SIRNA FOR INHIBITING GENE EXPRESSION OF LPA, COMPOSITION CONTAINING SAME, AND USE THEREOF**

(57) The present invention relates to an siRNA for inhibiting the gene expression of LPA, a composition containing same, and a use thereof. The siRNA comprises a sense strand and an antisense strand, wherein the antisense strand comprises a sequence complementary to an LPA mRNA sequence, and the sequence of the antisense strand includes a sequence of any antisense strand in Table 1.

1000
100
10
1
Lp(a) Level (%)
0
7
14
21
28
35
42
49
56
Days
PBS
GalNAC-SLN360 1mg/kg
JAB-190024-1 3mg/kg
JAB-190024-1 1mg/kg
JAB-190024-3 3mg/kg
JAB-190024-3 1mg/kg
JAB-190024-5 3mg/kg
JAB-190024-5 1mg/kg

FIG. 2

EP 4 796 633 A1

## Description

### TECHNICAL FIELD

The present application relates to the field of biomedicine, and specifically relates to siRNA for inhibiting gene expression of LPA, a composition comprising the same, and use thereof.

### BACKGROUND

LPA is the name of the gene encoding apolipoprotein(a) (apo(a)), which is mainly expressed in the liver, and the expression thereof is limited to humans and non-human primates. Apolipoprotein(a) is attached to apo(B)-100 via a disulfide bond and, combined with a lipid core, assembles into lipoprotein(a) (Lp(a)) particles. Lp(a) particles are cholesterol-rich, specialized macromolecular lipoproteins, the surfaces of which are encapsulated by cholesterol and phospholipids, and embedded with the hydrophilic apolipoprotein components apolipoprotein(a) and apo(B)-100. Lp(a) can enter and deposit on the vascular wall, having the effect of promoting atherosclerosis. Lp(a) is structurally homologous to plasminogen (PLG), and can compete with plasminogen for binding to fibrin sites, thereby inhibiting fibrinogen hydrolysis and promoting thrombosis. Therefore, Lp(a) is closely associated with atherosclerosis and thrombosis. Studies have shown that the level of Lp(a) in blood is an independent risk factor for cardiovascular disease, stroke, and atherosclerotic stenosis.

Analysis of Lp(a) levels in multiple studies suggests that a high Lp(a) level is an independent risk factor for cardiovascular disease, stroke, and other related disorders (including atherosclerotic stenosis). Furthermore, genome-wide association studies have also implicated LPA as a genetic risk factor for diseases such as atherosclerotic stenosis.

Therefore, there is a current need for methods for effectively treating and preventing disorders such as the following and associated ones, as well as reducing the risk of developing disorders such as the following and associated ones: stroke, atherosclerosis, thrombosis, and cardiovascular diseases such as coronary heart disease, aortic stenosis, and other currently unidentified related disorders, pathologies, or syndromes. The present application addresses this unmet medical need.

### SUMMARY

The purpose herein is to provide an siRNA for inhibiting the expression of the LPA gene (referred to herein as LPA siRNA) or a pharmaceutically acceptable salt thereof. The siRNA described herein, after being delivered to cells expressing the LPA gene, inhibits and/or knocks down the expression of LPA in vitro and/or in vivo through the biological process of RNA interference (RNAi).

Another purpose of the present application is to provide a pharmaceutical composition comprising the siRNA or the pharmaceutically acceptable salt thereof.

Another purpose of the present application is to provide use of the siRNA or the pharmaceutically acceptable salt thereof, or the pharmaceutical composition in preventing or treating a disease or disorder associated with LPA expression.

To achieve the above purposes, the present application provides an siRNA or a pharmaceutically acceptable salt thereof, comprising a sense strand and an antisense strand, wherein the antisense strand comprises a sequence complementary to an LPA mRNA sequence, and a sequence of the antisense strand comprises any one of the sequences of the antisense strands in Table 1:

**Table 1**

| siRNA name | Sense strand (5'-3') | SEQ ID NO: | Antisense strand (3'-5') | SEQ ID NO: |
|---|---|---|---|---|
| 190024001 | GUGGCAGCUCCUUAUUGUU | 1 | CACCGUCGAGGAAUAACAA | 128 |
| 190024002 | GCAGCUCCUUAUUGUUAUA | 2 | CGUCGAGGAAUAACAAUAU | 129 |
| 190024003 | GGCAGCUCCUUAUUGUUAU | 3 | CCGUCGAGGAAUAACAAUA | 130 |
| 190024004 | GCAAUGCUCAGACGCAGAA | 4 | CGUUACGAGUCUGCGUCUU | 131 |
| 190024005 | GCUUGAUCAAGAACUACUG | 5 | CGAACUAGUUCUUGAUGAC | 132 |
| 190024006 | CCUAGAGGCUCCUUCUGAA | 6 | GGAUCUCCGAGGAAGACUU | 133 |
| 190024007 | CUCCAAGCCUAGAGGCUUU | 7 | GAGGUUCGGAUCUCCGAAA | 134 |
| 190024008 | GAUGCUGAGAUUCGCCCUU | 8 | CUACGACUCUAAGCGGGAA | 135 |

(continued)

| siRNA name | Sense strand (5'-3') | SEQ ID NO: | Antisense strand (3'-5') | SEQ ID NO: |
|---|---|---|---|---|
| 190024009 | GCACGUACUCCACCACUGU | 9 | CGUGCAUGAGGUGGUGACA | 136 |
| 190024010 | GAAUCCAGAUGCUGAGAUU | 10 | CUUAGGUCUACGACUCUAA | 137 |
| 190024011 | UGGCAGCUCCUUAUUGUUA | 11 | ACCGUCGAGGAAUAACAAU | 138 |
| 190024012 | AGAGUUAUCGAGGCUCAUU | 12 | UCUCAAUAGCUCCGAGUAA | 139 |
| 190024013 | CCUUCUGAACAAGCACCAA | 13 | GGAAGACUUGUUCGUGGUU | 140 |
| 190024014 | ACUGUCACAGGAAGAACUU | 14 | UGACAGUGUCCUUCUUGAA | 141 |
| 190024015 | CCGAACAAGCACCGACUGA | 15 | GGCUUGUUCGUGGCUGACU | 142 |
| 190024016 | CCUAGAGGCUUUUUUUGAA | 16 | GGAUCUCCGAAAAAAACUU | 143 |
| 190024017 | CAAGCACCAACUGAGCAAA | 17 | GUUCGUGGUUGACUCGUUU | 144 |
| 190024018 | GGACCUGCCAAGCUUGGUC | 18 | CCUGGACGGUUCGAACCAG | 145 |
| 190024019 | ACCAUUAUGGACAGAGUUA | 19 | UGGUAAUACCUGUCUCAAU | 146 |
| 190024020 | GAAGCACCAACUGAAAACA | 20 | CUUCGUGGUUGACUUUUGU | 147 |
| 190024021 | AGGACUGCUACUACCAUUA | 21 | UCCUGACGAUGAUGGUAAU | 148 |
| 190024022 | GGCUCCUUCUGAACAAGCA | 22 | CCGAGGAAGACUUGUUCGU | 149 |
| 190024023 | AAUCCAGAUGCUGAGAUUA | 23 | UUAGGUCUACGACUCUAAU | 150 |
| 190024024 | CACUAUCACAGGAAGAACA | 24 | GUGAUAGUGUCCUUCUUGU | 151 |
| 190024025 | CAAGCACCGACUGAGCAAA | 25 | GUUCGUGGCUGACUCGUUU | 152 |
| 190024026 | GGCACGUACUCCACCACUG | 26 | CCGUGCAUGAGGUGGUGAC | 153 |
| 190024027 | GCUGAGAUUCGCCCUUGGU | 27 | CGACUCUAAGCGGGAACCA | 154 |
| 190024028 | GGCUCCUUCCGAACAAGCA | 28 | CCGAGGAAGGCUUGUUCGU | 155 |
| 190024029 | AGAAGCACCAACUGAAAAC | 29 | UCUUCGUGGUUGACUUUUG | 156 |
| 190024030 | CCUUCUGAAGAAGCACCAA | 30 | GGAAGACUUCUUCGUGGUU | 157 |
| 190024031 | UGUGGCAGCUCCUUAUUGU | 31 | ACACCGUCGAGGAAUAACA | 158 |
| 190024032 | GAGUUAUCGAGGCUCAUUC | 32 | CUCAAUAGCUCCGAGUAAG | 159 |
| 190024033 | GAGGCUUUUUUUGAACAAG | 33 | CUCCGAAAAAAACUUGUUC | 160 |
| 190024034 | GCCUAGAGGCUCCUUCUGA | 34 | CGGAUCUCCGAGGAAGACU | 161 |
| 190024035 | AAGAAGCACCAACUGAAAA | 35 | UUCUUCGUGGUUGACUUUU | 162 |
| 190024036 | ACAGGAAGAACAUGUCAGU | 36 | UGUCCUUCUUGUACAGUCA | 163 |
| 190024037 | GGCUGUUUCUGAACAAGCA | 37 | CCGACAAAGACUUGUUCGU | 164 |
| 190024038 | CGGUGGUCCCAGUUCCAAG | 38 | GCCACCAGGGUCAAGGUUC | 165 |
| 190024039 | AGGACCUGCCAAGCUUGGU | 39 | UCCUGGACGGUUCGAACCA | 166 |
| 190024040 | GUCGCUCCUCCGACUGUCA | 40 | CAGCGAGGAGGCUGACAGU | 167 |
| 190024041 | CAGGACUGCUACUACCAUU | 41 | GUCCUGACGAUGAUGGUAA | 168 |
| 190024042 | GCAUCAGAGGACAACAGAA | 42 | CGUAGUCUCCUGUUGUCUU | 169 |
| 190024043 | UGGCUUGAUCAAGAACUAC | 43 | ACCGAACUAGUUCUUGAUG | 170 |
| 190024044 | CACUGGCAUCAGAGGACAA | 44 | GUGACCGUAGUCUCCUGUU | 171 |
| 190024045 | AGCCUAGAGGCUCCUUCUG | 45 | UCGGAUCUCCGAGGAAGAC | 172 |
| 190024046 | GCACAGAGGCUUCUUCUGA | 46 | CGUGUCUCCGAAGAAGACU | 173 |
| 190024047 | CAGAAUCAGGUGUCCUAGA | 47 | GUCUUAGUCCACAGGAUCU | 174 |

(continued)

| siRNA name | Sense strand (5'-3') | SEQ ID NO: | Antisense strand (3'-5') | SEQ ID NO: |
|---|---|---|---|---|
| 190024048 | ACUAUCACAGGAAGAACAU | 48 | UGAUAGUGUCCUUCUUGUA | 175 |
| 190024049 | ACAGGACUGCUACUACCAU | 49 | UGUCCUGACGAUGAUGGUA | 176 |
| 190024050 | CUACCAUUAUGGACAGAGU | 50 | GAUGGUAAUACCUGUCUCA | 177 |
| 190024051 | GCCUAGAGGCUUUUUUUGA | 51 | CGGAUCUCCGAAAAAAACU | 178 |
| 190024052 | CCGCCAGGACUGAAUGUUA | 52 | GGCGGUCCUGACUUACAAU | 179 |
| 190024053 | CAAGCACCAACGGAGCAAA | 53 | GUUCGUGGUUGCCUCGUUU | 180 |
| 190024054 | CCCUUGGUGUUACACCAUG | 54 | GGGAACCACAAUGUGGUAC | 181 |
| 190024055 | CUGCUACUACCAUUAUGGA | 55 | GACGAUGAUGGUAAUACCU | 182 |
| 190024056 | ACAAGCACCACCUGAGAAA | 56 | UGUUCGUGGUGGACUCUUU | 183 |
| 190024057 | CAAUGCCUGGUGACAGAAU | 57 | GUUACGGACCACUGUCUUA | 184 |
| 190024058 | AAGCACCGACUGAGCAAAG | 58 | UUCGUGGCUGACUCGUUUC | 185 |
| 190024059 | CACAGAGGCUUCUUCUGAA | 59 | GUGUCUCCGAAGAAGACUU | 186 |
| 190024060 | GUGUCCUUGCAACUCUCAC | 60 | CACAGGAACGUUGAGAGUG | 187 |
| 190024061 | CAGCUCCUUAUUGUUAUAC | 61 | GUCGAGGAAUAACAAUAUG | 188 |
| 190024062 | GCUCCUUCCGAACAAGCAC | 62 | CGAGGAAGGCUUGUUCGUG | 189 |
| 190024063 | GGCAUCAGAGGACAACAGA | 63 | CCGUAGUCUCCUGUUGUCU | 190 |
| 190024064 | GCACCAACUGAAAACAGCA | 64 | CGUGGUUGACUUUUGUCGU | 191 |
| 190024065 | UGCUGAGAUUCGCCCUUGG | 65 | ACGACUCUAAGCGGGAACC | 192 |
| 190024066 | UUCCGAACAAGCACCGACU | 66 | AAGGCUUGUUCGUGGCUGA | 193 |
| 190024067 | GCUACUACCAUUAUGGACA | 67 | CGAUGAUGGUAAUACCUGU | 194 |
| 190024068 | GCACCAACUGAGCAAAGGC | 68 | CGUGGUUGACUCGUUUCCG | 195 |
| 190024069 | CAGGAAGAACAUGUCAGUC | 69 | GUCCUUCUUGUACAGUCAG | 196 |
| 190024070 | AAGCACCAACGGAGCAAAG | 70 | UUCGUGGUUGCCUCGUUUC | 197 |
| 190024071 | GACACCACACCAGCAUAGU | 71 | CUGUGGUGUGGUCGUAUCA | 198 |
| 190024072 | CUGUCACAGGAAGAACUUG | 72 | GACAGUGUCCUUCUUGAAC | 199 |
| 190024073 | CAGAGGACAACAGAAUAUU | 73 | GUCUCCUGUUGUCUUAUAA | 200 |
| 190024074 | CUGGCAUCAGAGGACAACA | 74 | GACCGUAGUCUCCUGUUGU | 201 |
| 190024075 | UACCAUUAUGGACAGAGUU | 75 | AUGGUAAUACCUGUCUCAA | 202 |
| 190024076 | UCAGAGGACAACAGAAUAU | 76 | AGUCUCCUGUUGUCUUAUA | 203 |
| 190024077 | CAUGGAUCCCAAUGUCAGA | 77 | GUACCUAGGGUUACAGUCU | 204 |
| 190024078 | CCAUUAUGGACAGAGUUAC | 78 | GGUAAUACCUGUCUCAAUG | 205 |
| 190024079 | AUACCAUGGAUCCCAAUGU | 79 | UAUGGUACCUAGGGUUACA | 206 |
| 190024080 | GCUCCUUCUGAACAAGCAC | 80 | CGAGGAAGACUUGUUCGUG | 207 |
| 190024081 | GCACCGACUGAGCAAAGGC | 81 | CGUGGCUGACUCGUUUCCG | 208 |
| 190024082 | UCCAAGCCUAGAGGCUUUU | 82 | AGGUUCGGAUCUCCGAAAA | 209 |
| 190024083 | UGACACCACACCAGCAUAG | 83 | ACUGUGGUGUGGUCGUAUC | 210 |
| 190024084 | CCCAGUUCCAAGCACAGAG | 84 | GGGUCAAGGUUCGUGUCUC | 211 |
| 190024085 | CACCACACCAGCAUAGUCG | 85 | GUGGUGUGGUCGUAUCAGC | 212 |
| 190024086 | GUGGUCCCAGUUCCAAGCA | 86 | CACCAGGGUCAAGGUUCGU | 213 |

(continued)

| siRNA name | Sense strand (5'-3') | SEQ ID NO: | Antisense strand (3'-5') | SEQ ID NO: |
|---|---|---|---|---|
| 190024087 | CAUCAGAGGACAACAGAAU | 87 | GUAGUCUCCUGUUGUCUUA | 214 |
| 190024088 | AAGCACAGAGGCUUCUUCU | 88 | UUCGUGUCUCCGAAGAAGA | 215 |
| 190024089 | GGUGGUCCCAGUUCCAAGC | 89 | CCACCAGGGUCAAGGUUCG | 216 |
| 190024090 | ACACCACACCAGCAUAGUC | 90 | UGUGGUGUGGUCGUAUCAG | 217 |
| 190024091 | AGCACCGACUGAGCAAAGG | 91 | UCGUGGCUGACUCGUUUCC | 218 |
| 190024092 | CUGACACGAUGUCCAGUGA | 92 | GACUGUGCUACAGGUCACU | 219 |
| 190024093 | GAUUCGCCCUUGGUGUUAC | 93 | CUAAGCGGGAACCACAAUG | 220 |
| 190024094 | CACCAUGGAUCCCAGUGUC | 94 | GUGGUACCUAGGGUCACAG | 221 |
| 190024095 | CCAAGCCUAGAGGCUUUUU | 95 | GGUUCGGAUCUCCGAAAAA | 222 |
| 190024096 | AGCACAGAGGCUUCUUCUG | 96 | UCGUGUCUCCGAAGAAGAC | 223 |
| 190024097 | CGGCUGUUUCUGAACAAGC | 97 | GCCGACAAAGACUUGUUCG | 224 |
| 190024098 | GUGGUCCCAGAUCCAAGCA | 98 | CACCAGGGUCUAGGUUCGU | 225 |
| 190024099 | CCAGUGACAGAAUCGAGUG | 99 | GGUCACUGUCUUAGCUCAC | 226 |
| 190024100 | AUCAGAGGACAACAGAAUA | 100 | UAGUCUCCUGUUGUCUUAU | 227 |
| 190024101 | AAGCACCAACUGAGCAAAG | 101 | UUCGUGGUUGACUCGUUUC | 228 |
| 190024102 | GAAGAACAUGUCAGUCUUG | 102 | CUUCUUGUACAGUCAGAAC | 229 |
| 190024103 | AAGCACCAACUGAAAACAG | 103 | UUCGUGGUUGACUUUUGUC | 230 |
| 190024104 | AGGCUCCUUCUGAACAAGC | 104 | UCCGAGGAAGACUUGUUCG | 231 |
| 190024105 | AGCACCAACUGAGCAAAGG | 105 | UCGUGGUUGACUCGUUUCC | 232 |
| 190024106 | UCCCACGGUGGUCCCAGUU | 106 | AGGGUGCCACCAGGGUCAA | 233 |
| 190024107 | UCCGAACAAGCACCGACUG | 107 | AGGCUUGUUCGUGGCUGAC | 234 |
| 190024108 | UCCACGGCUGUUUCUGAAC | 108 | AGGUGCCGACAAAGACUUG | 235 |
| 190024109 | GAAUCGAGUGUCCUCACAA | 109 | CUUAGCUCACAGGAGUGUU | 236 |
| 190024110 | CCUGACACGAUGUCCAGUG | 110 | GGACUGUGCUACAGGUCAC | 237 |
| 190024111 | UCCUCACAACUCCCACGGU | 111 | AGGAGUGUUGAGGGUGCCA | 238 |
| 190024112 | UGGCAUCAGAGGACAACAG | 112 | ACCGUAGUCUCCUGUUGUC | 239 |
| 190024113 | GGACAACAGAAUAUUAUCC | 113 | CCUGUUGUCUUAUAAUAGG | 240 |
| 190024114 | GGUGGUCCCAGAUCCAAGC | 114 | CCACCAGGGUCUAGGUUCG | 241 |
| 190024115 | AGGCUUCUUCUGAAGAAGC | 115 | UCCGAAGAAGACUUCUUCG | 242 |
| 190024116 | UCUCACGGUGGUCCCAGAU | 116 | AGAGUGCCACCAGGGUCUA | 243 |
| 190024117 | ACUGGCAUCAGAGGACAAC | 117 | UGACCGUAGUCUCCUGUUG | 244 |
| 190024118 | GGUGUUAUACCAUGGAUCC | 118 | CCACAAUAUGGUACCUAGG | 245 |
| 190024119 | ACACCAUGGAUCCCAGUGU | 119 | UGUGGUACCUAGGGUCACA | 246 |
| 190024120 | CUCACGGUGGUCCCAGAUC | 120 | GAGUGCCACCAGGGUCUAG | 247 |
| 190024121 | CCGGUUCCAAGCACAGAGG | 121 | GGCCAAGGUUCGUGUCUCC | 248 |
| 190024122 | CAAGCACAGAGGCUUCUUC | 122 | GUUCGUGUCUCCGAAGAAG | 249 |
| 190024123 | CCCUUGGUGUUAUACCAUG | 123 | GGGAACCACAAUAUGGUAC | 250 |
| 190024124 | UUGGUGUUAUACCAUGGAU | 124 | AACCACAAUAUGGUACCUA | 251 |
| 190024125 | GGCUUCUUCUGAAGAAGCA | 125 | CCGAAGAAGACUUCUUCGU | 252 |

(continued)

| siRNA name | Sense strand (5'-3') | SEQ ID NO: | Antisense strand (3'-5') | SEQ ID NO: |
|---|---|---|---|---|
| 190024126 | ACAGAGUUAUCGAGGCUCAUU | 126 | UGUCUCAAUAGCUCCGAGUAA | 253 |
| 190024127 | CAGAUGCUGAGAUUCGCCCUU | 127 | GUCUACGACUCUAAGCGGGAA | 254 |

In some embodiments, the antisense strand is 19 to 26 nucleobases in length.

In some embodiments, the antisense strand is 19 to 21 nucleobases in length.

In some embodiments, the sense strand comprises a sequence substantially complementary to the antisense strand, and forms a duplex complementary region of 15 to 26 contiguous base pairs.

In some embodiments, the duplex complementary region is 18 to 23 contiguous base pairs in length.

In some embodiments, the duplex complementary region is 19 to 21 contiguous base pairs in length.

In some embodiments, the sense strand is 19 to 26 nucleobases in length.

In some embodiments, the sense strand is 19 to 21 nucleobases in length.

In some embodiments, a sequence of the sense strand comprises any one of the sequences of the sense strands in Table 1.

In some embodiments, the antisense strand comprises any one of the sequences of SEQ ID NOs: 128-150 and 253-254.

In some embodiments, the sense strand comprises any one of the sequences of SEQ ID NOs: 1-23 and 126-127.

In some embodiments, the siRNA comprises any one of the siRNAs in Table 1.

In some embodiments, in the siRNA:

the antisense strand comprises SEQ ID NO: 128, and the sense strand comprises SEQ ID NO: 1;
the antisense strand comprises SEQ ID NO: 129, and the sense strand comprises SEQ ID NO: 2;
the antisense strand comprises SEQ ID NO: 130, and the sense strand comprises SEQ ID NO: 3;
the antisense strand comprises SEQ ID NO: 131, and the sense strand comprises SEQ ID NO: 4;
the antisense strand comprises SEQ ID NO: 132, and the sense strand comprises SEQ ID NO: 5;
the antisense strand comprises SEQ ID NO: 133, and the sense strand comprises SEQ ID NO: 6;
the antisense strand comprises SEQ ID NO: 134, and the sense strand comprises SEQ ID NO: 7;
the antisense strand comprises SEQ ID NO: 135, and the sense strand comprises SEQ ID NO: 8;
the antisense strand comprises SEQ ID NO: 136, and the sense strand comprises SEQ ID NO: 9;
the antisense strand comprises SEQ ID NO: 137, and the sense strand comprises SEQ ID NO: 10;
the antisense strand comprises SEQ ID NO: 138, and the sense strand comprises SEQ ID NO: 11;
the antisense strand comprises SEQ ID NO: 139, and the sense strand comprises SEQ ID NO: 12;
the antisense strand comprises SEQ ID NO: 140, and the sense strand comprises SEQ ID NO: 13;
the antisense strand comprises SEQ ID NO: 141, and the sense strand comprises SEQ ID NO: 14;
the antisense strand comprises SEQ ID NO: 142, and the sense strand comprises SEQ ID NO: 15;
the antisense strand comprises SEQ ID NO: 143, and the sense strand comprises SEQ ID NO: 16;
the antisense strand comprises SEQ ID NO: 144, and the sense strand comprises SEQ ID NO: 17;
the antisense strand comprises SEQ ID NO: 145, and the sense strand comprises SEQ ID NO: 18;
the antisense strand comprises SEQ ID NO: 146, and the sense strand comprises SEQ ID NO: 19;
the antisense strand comprises SEQ ID NO: 147, and the sense strand comprises SEQ ID NO: 20;
the antisense strand comprises SEQ ID NO: 148, and the sense strand comprises SEQ ID NO: 21;
the antisense strand comprises SEQ ID NO: 149, and the sense strand comprises SEQ ID NO: 22;
the antisense strand comprises SEQ ID NO: 150, and the sense strand comprises SEQ ID NO: 23;
the antisense strand comprises SEQ ID NO: 253, and the sense strand comprises SEQ ID NO: 126; or
the antisense strand comprises SEQ ID NO: 254, and the sense strand comprises SEQ ID NO: 127.

In some embodiments, the antisense strand or the sense strand contains 1, 2, 3, 4, 5, or 6 extensions.

In some embodiments, the extension of the antisense strand or the sense strand is dT.

In some embodiments, the antisense strand or the sense strand contains 2 dT extensions.

In some embodiments, the 3' end of the antisense strand contains two extended dTs, and the sense strand does not contain a dT.

In some embodiments, the antisense strand comprises any one of the sequences of the antisense strands in **Table 2**:

Table 2

| siRNA name | Sense strand (5'-3') | SEQ ID NO: | Antisense strand (3'-5') | SEQ ID NO: |
|---|---|---|---|---|
| 190024001dt | GUGGCAGCUCCUUAUUGUUdTdT | 255 | dTdTCACCGUCGAGGAAUAACAA | 380 |
| 190024002dt | GCAGCUCCUUAUUGUUAUAdTdT | 256 | dTdTCGUCGAGGAAUAACAAUAU | 381 |
| 190024003dt | GGCAGCUCCUUAUUGUUAUdTdT | 257 | dTdTCCGUCGAGGAAUAACAAUA | 382 |
| 190024004dt | GCAAUGCUCAGACGCAGAAdTdT | 258 | dTdTCGUUACGAGUCUGCGUCUU | 383 |
| 190024005dt | GCUUGAUCAAGAACUACUGdTdT | 259 | dTdTCGAACUAGUUCUUGAUGAC | 384 |
| 190024006dt | CCUAGAGGCUCCUUCUGAAdTdT | 260 | dTdTGGAUCUCCGAGGAAGACUU | 385 |
| 190024007dt | CUCCAAGCCUAGAGGCUUUdTdT | 261 | dTdTGAGGUUCGGAUCUCCGAAA | 386 |
| 190024008dt | GAUGCUGAGAUUCGCCCUUdTdT | 262 | dTdTCUACGACUCUAAGCGGGAA | 387 |
| 190024009dt | GCACGUACUCCACCACUGUdTdT | 263 | dTdTCGUGCAUGAGGUGGUGACA | 388 |
| 190024010dt | GAAUCCAGAUGCUGAGAUUdTdT | 264 | dTdTCUUAGGUCUACGACUCUAA | 389 |
| 190024011dt | UGGCAGCUCCUUAUUGUUAdTdT | 265 | dTdTACCGUCGAGGAAUAACAAU | 390 |
| 190024012dt | AGAGUUAUCGAGGCUCAUUdTdT | 266 | dTdTUCUCAAUAGCUCCGAGUAA | 391 |
| 190024013dt | CCUUCUGAACAAGCACCAAdTdT | 267 | dTdTGGAAGACUUGUUCGUGGUU | 392 |
| 190024014dt | ACUGUCACAGGAAGAACUUdTdT | 268 | dTdTUGACAGUGUCCUUCUUGAA | 393 |
| 190024015dt | CCGAACAAGCACCGACUGAdTdT | 269 | dTdTGGCUUGUUCGUGGCUGACU | 394 |
| 190024016dt | CCUAGAGGCUUUUUUUGAAdTdT | 270 | dTdTGGAUCUCCGAAAAAAACUU | 395 |
| 190024017dt | CAAGCACCAACUGAGCAAAdTdT | 271 | dTdTGUUCGUGGUUGACUCGUUU | 396 |
| 190024018dt | GGACCUGCCAAGCUUGGUCdTdT | 272 | dTdTCCUGGACGGUUCGAACCAG | 397 |
| 190024019dt | ACCAUUAUGGACAGAGUUAdTdT | 273 | dTdTUGGUAAUACCUGUCUCAAU | 398 |
| 190024020dt | GAAGCACCAACUGAAAACAdTdT | 274 | dTdTCUUCGUGGUUGACUUUUGU | 399 |
| 190024021dt | AGGACUGCUACUACCAUUAdTdT | 275 | dTdTUCCUGACGAUGAUGGUAAU | 400 |
| 190024022dt | GGCUCCUUCUGAACAAGCAdTdT | 276 | dTdTCCGAGGAAGACUUGUUCGU | 401 |
| 190024023dt | AAUCCAGAUGCUGAGAUUAdTdT | 277 | dTdTUUAGGUCUACGACUCUAAU | 402 |
| 190024024dt | CACUAUCACAGGAAGAACAdTdT | 278 | dTdTGUGAUAGUGUCCUUCUUGU | 403 |
| 190024025dt | CAAGCACCGACUGAGCAAAdTdT | 279 | dTdTGUUCGUGGCUGACUCGUUU | 404 |
| 190024026dt | GGCACGUACUCCACCACUGdTdT | 280 | dTdTCCGUGCAUGAGGUGGUGAC | 405 |
| 190024027dt | GCUGAGAUUCGCCCUUGGUdTdT | 281 | dTdTCGACUCUAAGCGGGAACCA | 406 |
| 190024028dt | GGCUCCUUCCGAACAAGCAdTdT | 282 | dTdTCCGAGGAAGGCUUGUUCGU | 407 |
| 190024029dt | AGAAGCACCAACUGAAAACdTdT | 283 | dTdTUCUUCGUGGUUGACUUUUG | 408 |
| 190024030dt | CCUUCUGAAGAAGCACCAAdTdT | 284 | dTdTGGAAGACUUCUUCGUGGUU | 409 |
| 190024031dt | UGUGGCAGCUCCUUAUUGUdTdT | 285 | dTdTACACCGUCGAGGAAUAACA | 410 |
| 190024032dt | GAGUUAUCGAGGCUCAUUCdTdT | 286 | dTdTCUCAAUAGCUCCGAGUAAG | 411 |
| 190024033dt | GAGGCUUUUUUUGAACAAGdTdT | 287 | dTdTCUCCGAAAAAAACUUGUUC | 412 |
| 190024034dt | GCCUAGAGGCUCCUUCUGAdTdT | 288 | dTdTCGGAUCUCCGAGGAAGACU | 413 |
| 190024035dt | AAGAAGCACCAACUGAAAAdTdT | 289 | dTdTUUCUUCGUGGUUGACUUUU | 414 |
| 190024036dt | ACAGGAAGAACAUGUCAGUdTdT | 290 | dTdTUGUCCUUCUUGUACAGUCA | 415 |
| 190024037dt | GGCUGUUUCUGAACAAGCAdTdT | 291 | dTdTCCGACAAAGACUUGUUCGU | 416 |
| 190024038dt | CGGUGGUCCCAGUUCCAAGdTdT | 292 | dTdTGCCACCAGGGUCAAGGUUC | 417 |
| 190024039dt | AGGACCUGCCAAGCUUGGUdTdT | 293 | dTdTUCCUGGACGGUUCGAACCA | 418 |

(continued)

| siRNA name | Sense strand (5'-3') | SEQ ID NO: | Antisense strand (3'-5') | SEQ ID NO: |
|---|---|---|---|---|
| 190024040dt | GUCGCUCCUCCGACUGUCAdTdT | 294 | dTdTCAGCGAGGAGGCUGACAGU | 419 |
| 190024041dt | CAGGACUGCUACUACCAUUdTdT | 295 | dTdTGUCCUGACGAUGAUGGUAA | 420 |
| 190024042dt | GCAUCAGAGGACAACAGAAdTdT | 296 | dTdTCGUAGUCUCCUGUUGUCUU | 421 |
| 190024043dt | UGGCUUGAUCAAGAACUACdTdT | 297 | dTdTACCGAACUAGUUCUUGAUG | 422 |
| 190024044dt | CACUGGCAUCAGAGGACAAdTdT | 298 | dTdTGUGACCGUAGUCUCCUGUU | 423 |
| 190024045dt | AGCCUAGAGGCUCCUUCUGdTdT | 299 | dTdTUCGGAUCUCCGAGGAAGAC | 424 |
| 190024046dt | GCACAGAGGCUUCUUCUGAdTdT | 300 | dTdTCGUGUCUCCGAAGAAGACU | 425 |
| 190024047dt | CAGAAUCAGGUGUCCUAGAdTdT | 301 | dTdTGUCUUAGUCCACAGGAUCU | 426 |
| 190024048dt | ACUAUCACAGGAAGAACAUdTdT | 302 | dTdTUGAUAGUGUCCUUCUUGUA | 427 |
| 190024049dt | ACAGGACUGCUACUACCAUdTdT | 303 | dTdTUGUCCUGACGAUGAUGGUA | 428 |
| 190024050dt | CUACCAUUAUGGACAGAGUdTdT | 304 | dTdTGAUGGUAAUACCUGUCUCA | 429 |
| 190024051dt | GCCUAGAGGCUUUUUUUGAdTdT | 305 | dTdTCGGAUCUCCGAAAAAAACU | 430 |
| 190024052dt | CCGCCAGGACUGAAUGUUAdTdT | 306 | dTdTGGCGGUCCUGACUUACAAU | 431 |
| 190024053dt | CAAGCACCAACGGAGCAAAdTdT | 307 | dTdTGUUCGUGGUUGCCUCGUUU | 432 |
| 190024054dt | CCCUUGGUGUUACACCAUGdTdT | 308 | dTdTGGGAACCACAAUGUGGUAC | 433 |
| 190024055dt | CUGCUACUACCAUUAUGGAdTdT | 309 | dTdTGACGAUGAUGGUAAUACCU | 434 |
| 190024056dt | ACAAGCACCACCUGAGAAAdTdT | 310 | dTdTUGUUCGUGGUGGACUCUUU | 435 |
| 190024057dt | CAAUGCCUGGUGACAGAAUdTdT | 311 | dTdTGUUACGGACCACUGUCUUA | 436 |
| 190024058dt | AAGCACCGACUGAGCAAAGdTdT | 312 | dTdTUUCGUGGCUGACUCGUUUC | 437 |
| 190024059dt | CACAGAGGCUUCUUCUGAAdTdT | 313 | dTdTGUGUCUCCGAAGAAGACUU | 438 |
| 190024060dt | GUGUCCUUGCAACUCUCACdTdT | 314 | dTdTCACAGGAACGUUGAGAGUG | 439 |
| 190024061dt | CAGCUCCUUAUUGUUAUACdTdT | 315 | dTdTGUCGAGGAAUAACAAUAUG | 440 |
| 190024062dt | GCUCCUUCCGAACAAGCACdTdT | 316 | dTdTCGAGGAAGGCUUGUUCGUG | 441 |
| 190024063dt | GGCAUCAGAGGACAACAGAdTdT | 317 | dTdTCCGUAGUCUCCUGUUGUCU | 442 |
| 190024064dt | GCACCAACUGAAAACAGCAdTdT | 318 | dTdTCGUGGUUGACUUUUGUCGU | 443 |
| 190024065dt | UGCUGAGAUUCGCCCUUGGdTdT | 319 | dTdTACGACUCUAAGCGGGAACC | 444 |
| 190024066dt | UUCCGAACAAGCACCGACUdTdT | 320 | dTdTAAGGCUUGUUCGUGGCUGA | 445 |
| 190024067dt | GCUACUACCAUUAUGGACAdTdT | 321 | dTdTCGAUGAUGGUAAUACCUGU | 446 |
| 190024068dt | GCACCAACUGAGCAAAGGCdTdT | 322 | dTdTCGUGGUUGACUCGUUUCCG | 447 |
| 190024069dt | CAGGAAGAACAUGUCAGUCdTdT | 323 | dTdTGUCCUUCUUGUACAGUCAG | 448 |
| 190024070dt | AAGCACCAACGGAGCAAAGdTdT | 324 | dTdTUUCGUGGUUGCCUCGUUUC | 449 |
| 190024071dt | GACACCACACCAGCAUAGUdTdT | 325 | dTdTCUGUGGUGUGGUCGUAUCA | 450 |
| 190024072dt | CUGUCACAGGAAGAACUUGdTdT | 326 | dTdTGACAGUGUCCUUCUUGAAC | 451 |
| 190024073dt | CAGAGGACAACAGAAUAUUdTdT | 327 | dTdTGUCUCCUGUUGUCUUAUAA | 452 |
| 190024074dt | CUGGCAUCAGAGGACAACAdTdT | 328 | dTdTGACCGUAGUCUCCUGUUGU | 453 |
| 190024075dt | UACCAUUAUGGACAGAGUUdTdT | 329 | dTdTAUGGUAAUACCUGUCUCAA | 454 |
| 190024076dt | UCAGAGGACAACAGAAUAUdTdT | 330 | dTdTAGUCUCCUGUUGUCUUAUA | 455 |
| 190024077dt | CAUGGAUCCCAAUGUCAGAdTdT | 331 | dTdTGUACCUAGGGUUACAGUCU | 456 |
| 190024078dt | CCAUUAUGGACAGAGUUACdTdT | 332 | dTdTGGUAAUACCUGUCUCAAUG | 457 |

(continued)

| siRNA name | Sense strand (5'-3') | SEQ ID NO: | Antisense strand (3'-5') | SEQ ID NO: |
|---|---|---|---|---|
| 190024079dt | AUACCAUGGAUCCCAAUGUdTdT | 333 | dTdTUAUGGUACCUAGGGUUACA | 458 |
| 190024080dt | GCUCCUUCUGAACAAGCACdTdT | 334 | dTdTCGAGGAAGACUUGUUCGUG | 459 |
| 190024081dt | GCACCGACUGAGCAAAGGCdTdT | 335 | dTdTCGUGGCUGACUCGUUUCCG | 460 |
| 190024082dt | UCCAAGCCUAGAGGCUUUUdTdT | 336 | dTdTAGGUUCGGAUCUCCGAAAA | 461 |
| 190024083dt | UGACACCACACCAGCAUAGdTdT | 337 | dTdTACUGUGGUGUGGUCGUAUC | 462 |
| 190024084dt | CCCAGUUCCAAGCACAGAGdTdT | 338 | dTdTGGGUCAAGGUUCGUGUCUC | 463 |
| 190024085dt | CACCACACCAGCAUAGUCGdTdT | 339 | dTdTGUGGUGUGGUCGUAUCAGC | 464 |
| 190024086dt | GUGGUCCCAGUUCCAAGCAdTdT | 340 | dTdTCACCAGGGUCAAGGUUCGU | 465 |
| 190024087dt | CAUCAGAGGACAACAGAAUdTdT | 341 | dTdTGUAGUCUCCUGUUGUCUUA | 466 |
| 190024088dt | AAGCACAGAGGCUUCUUCUdTdT | 342 | dTdTUUCGUGUCUCCGAAGAAGA | 467 |
| 190024089dt | GGUGGUCCCAGUUCCAAGCdTdT | 343 | dTdTCCACCAGGGUCAAGGUUCG | 468 |
| 190024090dt | ACACCACACCAGCAUAGUCdTdT | 344 | dTdTUGUGGUGUGGUCGUAUCAG | 469 |
| 190024091dt | AGCACCGACUGAGCAAAGGdTdT | 345 | dTdTUCGUGGCUGACUCGUUUCC | 470 |
| 190024092dt | CUGACACGAUGUCCAGUGAdTdT | 346 | dTdTGACUGUGCUACAGGUCACU | 471 |
| 190024093dt | GAUUCGCCCUUGGUGUUACdTdT | 347 | dTdTCUAAGCGGGAACCACAAUG | 472 |
| 190024094dt | CACCAUGGAUCCCAGUGUCdTdT | 348 | dTdTGUGGUACCUAGGGUCACAG | 473 |
| 190024095dt | CCAAGCCUAGAGGCUUUUUdTdT | 349 | dTdTGGUUCGGAUCUCCGAAAAA | 474 |
| 190024096dt | AGCACAGAGGCUUCUUCUGdTdT | 350 | dTdTUCGUGUCUCCGAAGAAGAC | 475 |
| 190024097dt | CGGCUGUUUCUGAACAAGCdTdT | 351 | dTdTGCCGACAAAGACUUGUUCG | 476 |
| 190024098dt | GUGGUCCCAGAUCCAAGCAdTdT | 352 | dTdTCACCAGGGUCUAGGUUCGU | 477 |
| 190024099dt | CCAGUGACAGAAUCGAGUGdTdT | 353 | dTdTGGUCACUGUCUUAGCUCAC | 478 |
| 190024100dt | AUCAGAGGACAACAGAAUAdTdT | 354 | dTdTUAGUCUCCUGUUGUCUUAU | 479 |
| 190024101dt | AAGCACCAACUGAGCAAAGdTdT | 355 | dTdTUUCGUGGUUGACUCGUUUC | 480 |
| 190024102dt | GAAGAACAUGUCAGUCUUGdTdT | 356 | dTdTCUUCUUGUACAGUCAGAAC | 481 |
| 190024103dt | AAGCACCAACUGAAAACAGdTdT | 357 | dTdTUUCGUGGUUGACUUUUGUC | 482 |
| 190024104dt | AGGCUCCUUCUGAACAAGCdTdT | 358 | dTdTUCCGAGGAAGACUUGUUCG | 483 |
| 190024105dt | AGCACCAACUGAGCAAAGGdTdT | 359 | dTdTUCGUGGUUGACUCGUUUCC | 484 |
| 190024106dt | UCCCACGGUGGUCCCAGUUdTdT | 360 | dTdTAGGGUGCCACCAGGGUCAA | 485 |
| 190024107dt | UCCGAACAAGCACCGACUGdTdT | 361 | dTdTAGGCUUGUUCGUGGCUGAC | 486 |
| 190024108dt | UCCACGGCUGUUUCUGAACdTdT | 362 | dTdTAGGUGCCGACAAAGACUUG | 487 |
| 190024109dt | GAAUCGAGUGUCCUCACAAdTdT | 363 | dTdTCUUAGCUCACAGGAGUGUU | 488 |
| 190024110dt | CCUGACACGAUGUCCAGUGdTdT | 364 | dTdTGGACUGUGCUACAGGUCAC | 489 |
| 190024111dt | UCCUCACAACUCCCACGGUdTdT | 365 | dTdTAGGAGUGUUGAGGGUGCCA | 490 |
| 190024112dt | UGGCAUCAGAGGACAACAGdTdT | 366 | dTdTACCGUAGUCUCCUGUUGUC | 491 |
| 190024113dt | GGACAACAGAAUAUUAUCCdTdT | 367 | dTdTCCUGUUGUCUUAUAAUAGG | 492 |
| 190024114dt | GGUGGUCCCAGAUCCAAGCdTdT | 368 | dTdTCCACCAGGGUCUAGGUUCG | 493 |
| 190024115dt | AGGCUUCUUCUGAAGAAGCdTdT | 369 | dTdTUCCGAAGAAGACUUCUUCG | 494 |
| 190024116dt | UCUCACGGUGGUCCCAGAUdTdT | 370 | dTdTAGAGUGCCACCAGGGUCUA | 495 |
| 190024117dt | ACUGGCAUCAGAGGACAACdTdT | 371 | dTdTUGACCGUAGUCUCCUGUUG | 496 |

(continued)

| siRNA name | Sense strand (5'-3') | SEQ ID NO: | Antisense strand (3'-5') | SEQ ID NO: |
|---|---|---|---|---|
| 190024118dt | GGUGUUAUACCAUGGAUCCdTdT | 372 | dTdTCCACAAUAUGGUACCUAGG | 497 |
| 190024119dt | ACACCAUGGAUCCCAGUGUdTdT | 373 | dTdTUGUGGUACCUAGGGUCACA | 498 |
| 190024120dt | CUCACGGUGGUCCCAGAUCdTdT | 374 | dTdTGAGUGCCACCAGGGUCUAG | 499 |
| 190024121dt | CCGGUUCCAAGCACAGAGGdTdT | 375 | dTdTGGCCAAGGUUCGUGUCUCC | 500 |
| 190024122dt | CAAGCACAGAGGCUUCUUCdTdT | 376 | dTdTGUUCGUGUCUCCGAAGAAG | 501 |
| 190024123dt | CCCUUGGUGUUAUACCAUGdTdT | 377 | dTdTGGGAACCACAAUAUGGUAC | 502 |
| 190024124dt | UUGGUGUUAUACCAUGGAUdTdT | 378 | dTdTAACCACAAUAUGGUACCUA | 503 |
| 190024125dt | GGCUUCUUCUGAAGAAGCAdTdT | 379 | dTdTCCGAAGAAGACUUCUUCGU | 504 |
| 190024001dt1 | GUGGCAGCUCCUUAUUGUU | 541 | dTdTCACCGUCGAGGAAUAACAA | 544 |
| 190024002dt1 | GCAGCUCCUUAUUGUUAUA | 542 | dTdTCGUCGAGGAAUAACAAUAU | 545 |
| 190024005dt1 | GCUUGAUCAAGAACUACUG | 543 | dTdTCGAACUAGUUCUUGAUGAC | 546 |

In some embodiments, the antisense strand comprises any one of the sequences of SEQ ID NOs: 380-402 and 544-546.

In some embodiments, the sense strand comprises any one of the sequences of the sense strands in Table 2.

In some embodiments, the sense strand comprises any one of the sequences of SEQ ID NOs: 255-277 and 541-543.

In some embodiments, the siRNA comprises any one of the siRNAs in Table 2.

In some embodiments, in the siRNA:

the antisense strand comprises SEQ ID NO: 380, and the sense strand comprises SEQ ID NO: 255;
the antisense strand comprises SEQ ID NO: 381, and the sense strand comprises SEQ ID NO: 256;
the antisense strand comprises SEQ ID NO: 382, and the sense strand comprises SEQ ID NO: 257;
the antisense strand comprises SEQ ID NO: 383, and the sense strand comprises SEQ ID NO: 258;
the antisense strand comprises SEQ ID NO: 384, and the sense strand comprises SEQ ID NO: 259;
the antisense strand comprises SEQ ID NO: 385, and the sense strand comprises SEQ ID NO: 260;
the antisense strand comprises SEQ ID NO: 386, and the sense strand comprises SEQ ID NO: 261;
the antisense strand comprises SEQ ID NO: 387, and the sense strand comprises SEQ ID NO: 262;
the antisense strand comprises SEQ ID NO: 388, and the sense strand comprises SEQ ID NO: 263;
the antisense strand comprises SEQ ID NO: 389, and the sense strand comprises SEQ ID NO: 264;
the antisense strand comprises SEQ ID NO: 390, and the sense strand comprises SEQ ID NO: 265;
the antisense strand comprises SEQ ID NO: 391, and the sense strand comprises SEQ ID NO: 266;
the antisense strand comprises SEQ ID NO: 392, and the sense strand comprises SEQ ID NO: 267;
the antisense strand comprises SEQ ID NO: 393, and the sense strand comprises SEQ ID NO: 268;
the antisense strand comprises SEQ ID NO: 394, and the sense strand comprises SEQ ID NO: 269;
the antisense strand comprises SEQ ID NO: 395, and the sense strand comprises SEQ ID NO: 270;
the antisense strand comprises SEQ ID NO: 396, and the sense strand comprises SEQ ID NO: 271;
the antisense strand comprises SEQ ID NO: 397, and the sense strand comprises SEQ ID NO: 272;
the antisense strand comprises SEQ ID NO: 398, and the sense strand comprises SEQ ID NO: 273;
the antisense strand comprises SEQ ID NO: 399, and the sense strand comprises SEQ ID NO: 274;
the antisense strand comprises SEQ ID NO: 400, and the sense strand comprises SEQ ID NO: 275;
the antisense strand comprises SEQ ID NO: 401, and the sense strand comprises SEQ ID NO: 276;
the antisense strand comprises SEQ ID NO: 402, and the sense strand comprises SEQ ID NO: 277;
the antisense strand comprises SEQ ID NO: 541, and the sense strand comprises SEQ ID NO: 544;
the antisense strand comprises SEQ ID NO: 542, and the sense strand comprises SEQ ID NO: 545; or
the antisense strand comprises SEQ ID NO: 543, and the sense strand comprises SEQ ID NO: 546.

In some embodiments, the sense strand and/or the antisense strand independently comprise one or more modified nucleotides.

In some embodiments, the modified nucleotides are 2'-modified nucleotides.

In some embodiments, the modified nucleotides comprise: 2'-O-methyl modified nucleotides (i.e., ribonucleotides in which the 2'-OH on the ribose is replaced by 2'-OMe, denoted herein as mN, where N represents a nucleotide), 2'-O-methoxyethyl modified nucleotides, 2'-fluoro modified nucleotides (i.e., ribonucleotides in which the 2'-OH on the ribose is replaced by 2'-F, denoted herein as fN, where N represents a ribonucleotide, also denoted herein as 2'-deoxy-2'-fluoro modified nucleotides), 2'-deoxy modified nucleotides, 2'-O-alkenyl modified nucleotides, locked nucleotides, GNA, LNA, abasic nucleotides, deoxythymidine, inverted deoxythymidine, deoxyadenosine, inverted deoxyadenosine, 2'-amino-modified nucleotides, 2'-alkyl-modified nucleotides, morpholino nucleotides, nucleotides comprising non-natural bases, 5'-(E)-vinylphosphonate modified nucleotides (denoted herein as VP), or a combination thereof.

In some embodiments, the nucleotides are all modified nucleotides.

In some embodiments, the modified nucleotides comprise 2'-O-methyl modified nucleotides, 2'-fluoro modified nucleotides, deoxythymidine, and 5'-(E)-vinylphosphonate modified nucleotides, or a combination thereof.

In some embodiments, the sense strand, the antisense strand, or both the sense strand and the antisense strand comprise one or more phosphorothioate or phosphorodithioate internucleoside linkages.

In some embodiments, the antisense strand comprises two consecutive phosphorothioate internucleoside linkages between terminal nucleotides at the 3' end and between terminal nucleotides at the 5' end.

In some embodiments, the sense strand comprises two consecutive phosphorothioate internucleoside linkages between terminal nucleotides at the 3' end and between terminal nucleotides at the 5' end.

In some embodiments, the sense strand comprises two consecutive phosphorothioate internucleoside linkages between terminal nucleotides at the 5' end, and the sense strand comprises one phosphorothioate internucleoside linkage between terminal nucleotides at the 3' end.

In some embodiments, the sense strand comprises two consecutive phosphorothioate internucleoside linkages between terminal nucleotides at the 5' end, and the sense strand comprises one phosphorothioate internucleoside linkage between terminal nucleotides at the 3' end; and the antisense strand comprises two consecutive phosphorothioate internucleoside linkages between the terminal nucleotides at the 3' end and the 5' end.

In some embodiments:

1) the sense strand comprises 21 nucleotides, the antisense strand comprises 23 nucleotides, the duplex complementary region is 21 base pairs in length, nucleotides at positions 9, 10, and 11 from the 5' end of the sense strand are all 2'-fluoro modified nucleotides, and other nucleotides of the sense strand are all 2'-O-methyl modified nucleotides; nucleotides at positions 8, 10, 18, and 22 from the 3' end of the antisense strand are all 2'-fluoro modified nucleotides, and other nucleotides of the antisense strand are all 2'-O-methyl modified nucleotides; a linkage between nucleotides at positions 1 to 3 from the 5' end of the sense strand, between nucleotides at positions 1 to 3 from the 3' end of the sense strand, between nucleotides at positions 1 to 3 from the 5' end of the antisense strand, and between nucleotides at positions 1 to 3 from the 3' end of the antisense strand is a phosphorothioate linkage; and a linkage between other nucleotides of the sense strand and between other nucleotides of the antisense strand is a phosphodiester linkage;

2) the sense strand comprises 19 nucleotides, the antisense strand comprises 21 nucleotides, the duplex complementary region is 19 base pairs in length, nucleotides at positions 7, 8, and 9 from the 5' end of the sense strand are all 2'-fluoro modified nucleotides, and other nucleotides of the sense strand are all 2'-O-methyl modified nucleotides; positions 1 and 2 from the 3' end of the antisense strand are two dTs, nucleotides at positions 6, 8, 14, 16, and 20 from the 3' end of the antisense strand are all 2'-fluoro modified nucleotides, and other nucleotides of the antisense strand are all 2'-O-methyl modified nucleotides; a linkage between nucleotides at positions 1 to 3 from the 5' end of the sense strand, between nucleotides at positions 1 to 3 from the 3' end of the sense strand, between nucleotides at positions 1 to 3 from the 5' end of the antisense strand, and between nucleotides at positions 1 to 3 from the 3' end of the antisense strand is a phosphorothioate linkage; and a linkage between other nucleotides of the sense strand and between other nucleotides of the antisense strand is a phosphodiester linkage;

3) the sense strand comprises 19 nucleotides, the antisense strand comprises 21 nucleotides, the duplex complementary region is 19 base pairs in length, nucleotides at positions 7, 8, and 9 from the 5' end of the sense strand are all 2'-fluoro modified nucleotides, and other nucleotides of the sense strand are all 2'-O-methyl modified nucleotides; positions 1 and 2 from the 3' end of the antisense strand are two dTs, nucleotides at positions 4, 6, 8, 16, and 20 from the 3' end of the antisense strand are all 2'-fluoro modified nucleotides, and other nucleotides of the antisense strand are all 2'-O-methyl modified nucleotides; a linkage between nucleotides at positions 1 to 3 from the 5' end of the sense strand, between nucleotides at positions 1 to 3 from the 3' end of the sense strand, between nucleotides at positions 1 to 3 from the 5' end of the antisense strand, and between nucleotides at positions 1 to 3 from the 3' end of the antisense strand is a phosphorothioate linkage; and a linkage between other nucleotides of the sense strand and between other nucleotides of the antisense strand is a phosphodiester linkage;

4) the sense strand comprises 21 nucleotides, the antisense strand comprises 21 nucleotides, the duplex com-

plementary region is 21 base pairs in length, nucleotides at positions 9, 10, and 11 from the 5' end of the sense strand are all 2'-fluoro modified nucleotides, and other nucleotides of the sense strand are all 2'-O-methyl modified nucleotides; nucleotides at positions 4, 6, 8, 10, 16, 18, and 20 starting from position 3 from the 3' end of the antisense strand are all 2'-fluoro modified nucleotides, and other nucleotides of the antisense strand are 2'-O-methyl modified nucleotides; a linkage between nucleotides at positions 1 to 3 from the 5' end of the sense strand, between nucleotides at positions 1 to 3 from the 3' end of the sense strand, between nucleotides at positions 1 to 3 from the 5' end of the antisense strand, and between nucleotides at positions 1 to 3 from the 3' end of the antisense strand is a phosphorothioate linkage; and a linkage between other nucleotides of the sense strand and between other nucleotides of the antisense strand is a phosphodiester linkage;

5) the sense strand comprises 21 nucleotides, the antisense strand comprises 21 nucleotides, the duplex complementary region is 21 base pairs in length, nucleotides at positions 9, 10, and 11 from the 5' end of the sense strand are all 2'-fluoro modified nucleotides, and other nucleotides of the sense strand are all 2'-O-methyl modified nucleotides; nucleotides at positions 4, 6, 8, 10, 16, 18, and 20 starting from position 3 from the 3' end of the antisense strand are all 2'-fluoro modified nucleotides, other nucleotides of the antisense strand are 2'-O-methyl modified nucleotides, and nucleotide at position 1 from the 5' end of the antisense strand is modified with VP; a linkage between nucleotides at positions 1 to 3 from the 5' end of the sense strand, between nucleotides at positions 1 to 3 from the 3' end of the sense strand, between nucleotides at positions 1 to 3 from the 5' end of the antisense strand, and between nucleotides at positions 1 to 3 from the 3' end of the antisense strand is a phosphorothioate linkage; and a linkage between other nucleotides of the sense strand and between other nucleotides of the antisense strand is a phosphodiester linkage;

6) the sense strand comprises 19 nucleotides, the antisense strand comprises 21 nucleotides, the duplex complementary region is 19 base pairs in length, nucleotides at positions 7, 8, and 9 from the 5' end of the sense strand are all 2'-fluoro modified nucleotides, and other nucleotides of the sense strand are all 2'-O-methyl modified nucleotides; positions 1 and 2 from the 3' end of the antisense strand are two dTs, nucleotides at positions 6, 8, 14, 16, and 20 from the 3' end of the antisense strand are all 2'-fluoro modified nucleotides, other nucleotides of the antisense strand are all 2'-O-methyl modified nucleotides, and nucleotide at position 1 from the 5' end of the antisense strand is modified with VP; a linkage between nucleotides at positions 1 to 3 from the 5' end of the sense strand, between nucleotides at positions 1 to 3 from the 3' end of the sense strand, between nucleotides at positions 1 to 3 from the 5' end of the antisense strand, and between nucleotides at positions 1 to 3 from the 3' end of the antisense strand is a phosphorothioate linkage; and a linkage between other nucleotides of the sense strand and between other nucleotides of the antisense strand is a phosphodiester linkage;

7) the sense strand comprises 19 nucleotides, the antisense strand comprises 21 nucleotides, the duplex complementary region is 19 base pairs in length, nucleotides at positions 7, 8, and 9 from the 5' end of the sense strand are all 2'-fluoro modified nucleotides, and other nucleotides of the sense strand are all 2'-O-methyl modified nucleotides; positions 1 and 2 from the 3' end of the antisense strand are two dTs, nucleotides at positions 6, 8, 14, 16, and 20 from the 3' end of the antisense strand are all 2'-fluoro modified nucleotides, and other nucleotides of the antisense strand are all 2'-O-methyl modified nucleotides; a linkage between nucleotides at positions 1 to 3 from the 5' end of the sense strand, between nucleotides at positions 1 to 2 from the 3' end of the sense strand, between nucleotides at positions 1 to 3 from the 5' end of the antisense strand, and between nucleotides at positions 1 to 3 from the 3' end of the antisense strand is a phosphorothioate linkage; and a linkage between other nucleotides of the sense strand and between other nucleotides of the antisense strand is a phosphodiester linkage; or

8) the sense strand comprises 19 nucleotides, the antisense strand comprises 21 nucleotides, the duplex complementary region is 19 base pairs in length, nucleotides at positions 7, 8, and 9 from the 5' end of the sense strand are all 2'-fluoro modified nucleotides, and other nucleotides of the sense strand are all 2'-O-methyl modified nucleotides; positions 1 and 2 from the 3' end of the antisense strand are two dTs, nucleotides at positions 6, 8, 14, 16, and 20 from the 3' end of the antisense strand are all 2'-fluoro modified nucleotides, other nucleotides of the antisense strand are all 2'-O-methyl modified nucleotides, and nucleotide at position 1 from the 5' end of the antisense strand is modified with VP; a linkage between nucleotides at positions 1 to 3 from the 5' end of the sense strand, between nucleotides at positions 1 to 2 from the 3' end of the sense strand, between nucleotides at positions 1 to 3 from the 5' end of the antisense strand, and between nucleotides at positions 1 to 3 from the 3' end of the antisense strand is a phosphorothioate linkage; and a linkage between other nucleotides of the sense strand and between other nucleotides of the antisense strand is a phosphodiester linkage.

In some embodiments, the antisense strand comprises any one of the antisense strands in **Table 3**:

Table 3

| siRNA name | Single strand | Sequence | SEQ ID NO: |
|---|---|---|---|
| 190024-M1 | antisense strand (5'-3') | mU*fA*mUmAmAfCmAmAmUmAmAmGmGfAmGfCmUmGmCmCmA*mC*mA | 505 |
| | sense strand (5'-3') | mU*mG*mGmCmAmGmCmUfCfCfUmUmAmUmUmGmUmUmA*mU*mA | 506 |
| 190024-M2 | antisense strand (3'-5') | dT*dT*mCmGmUfCmGfAmGmGmAmAmUfAmAfCmAmAmU*fA*mU | 507 |
| | sense strand (5'-3') | mG*mC*mAmGmCmUfCfCfUmUmAmUmUmGmUmUmA*mU*mA | 508 |
| 190024-M3 | antisense strand (3'-5') | dT*dT*mCmGmUfCmGfAmGmGmAmAmUfAmAfCmAmAmU*fA*mU | 509 |
| | sense strand (5'-3') | mG*mC*mAmGmCmUfCfCfUmUmAmUmUmGmUmUmAmU*mA | 510 |
| 190024-M4 | antisense strand (3'-5') | dT*dT*mCmGmUfCmGfAmGmGmAmAmUfAmAfCmAmAmU*fA*mUVP | 511 |
| | sense strand (5'-3') | mG*mC*mAmGmCmUfCfCfUmUmAmUmUmGmUmUmAmU*mA | 512 |
| 190024-M5 | antisense strand (3'-5') | dT*dT*mCmGmUfCmGfAmGmGmAmAmUfAmAfCmAmAmU*fA*mUVP | 513 |
| | sense strand (5'-3') | mG*mC*mAmGmCmUfCfCfUmUmAmUmUmGmUmUmA*mU*mA | 514 |
| 190024-M6 | antisense strand (3'-5') | dT*dT*mCmGmAfAmCfUmAmGmUmUmCfUmUfGmAmUmG*fA*mC | 515 |
| | sense strand (5'-3') | mG*mC*mUmUmGmAfUfCfAmAmGmAmAmCmUmAmC*mU*mG | 516 |
| 190024-M7 | antisense strand (3'-5') | dT*dT*mCmAmCfCmGfUmCmGmAmGmGfAmAfUmAmAmC*fA*mA | 517 |
| | sense strand (5'-3') | mG*mU*mGmGmCmAfGfCfUmCmCmUmUmAmUmUmG*mU*mU | 518 |

(continued)

| siRNA name | Single strand | Sequence | SEQ ID NO: |
|---|---|---|---|
| 190024-M8 | antisense strand (3'-5') | dT*dT*mCfGmUfCmGfAmGmGmAmAmUmAmAfCmAmA mU*fA*mU | 519 |
| | sense strand (5'-3') | mG*mC*mAmGmCmUfCfCfUmUmAmUmUmGmUmUmA *mU*mA | 520 |
| 190024-M9 | antisense strand (3'-5') | mU*mG*mUfCmUfCmAfAmUfAmGmCmUmCmCfGmAfG mU*fA*mA | 521 |
| | sense strand (5'-3') | mA*mC*mAmGmAmGmUmUfAfUfCmGmAmGmGmCmU mCmA*mU*mU | 522 |
| 190024-M10 | antisense strand (3'-5') | mG*mU*mCfUmAfCmGfAmCfUmCmUmAmAmGfCmGfG mG*fA*mA | 523 |
| | sense strand (5'-3') | mC*mA*mGmAmUmGmCmUfGfAfGmAmUmUmCmGmC mCmC*mU*mU | 524 |
| 190024-M11 | antisense strand (3'-5') | mG*mU*mCfUmAfCmGfAmCfUmCmUmAmAmGfCmGfG mG*fA*mAVP | 525 |
| | sense strand (5'-3') | mC*mA*mGmAmUmGmCmUfGfAfGmAmUmUmCmGmC mCmC*mU*mU | 526 |
| 190024-M12 | antisense strand (3'-5') | mU*mG*mUfCmUfCmAfAmUfAmGmCmUmCmCfGmAfG mU*fA*mAVP | 527 |
| | sense strand (5'-3') | mA*mC*mAmGmAmGmUmUfAfUfCmGmAmGmGmCmU mCmA*mU*mU | 528 |

In Table 3, fA, fC, fU, and fG represent 2'-fluoro modified A, C, U, and G ribonucleoside-3'-phosphates, respectively; mA, mC, mU, and mG represent 2'-O-methyl modified A, C, U, and G ribonucleoside-3'-phosphates, respectively; * represents a phosphorothioate linkage; dT represents thymidine deoxyribonucleoside-3'-phosphate; and VP represents a 5'-(E)-vinylphosphonate group-modified ribonucleotide.

In some embodiments, the antisense strand comprises any one of the antisense strands of SEQ ID NOs: 505, 507, 509, 511, 513, 515, 517, 519, 521, 523, 525, and 527.

In some embodiments, the sense strand comprises any one of the sense strands in Table 3.

In some embodiments, the sense strand comprises any one of the sense strands of SEQ ID NOs: 506, 508, 510, 512, 514, 516, 518, 520, 522, 524, 526, and 528.

In some embodiments, the siRNA comprises any one of the siRNAs in Table 3.

In some embodiments, in the siRNA:

the antisense strand comprises SEQ ID NO: 505, and the sense strand comprises SEQ ID NO: 506;
the antisense strand comprises SEQ ID NO: 507, and the sense strand comprises SEQ ID NO: 508;
the antisense strand comprises SEQ ID NO: 509, and the sense strand comprises SEQ ID NO: 510;
the antisense strand comprises SEQ ID NO: 511, and the sense strand comprises SEQ ID NO: 512;
the antisense strand comprises SEQ ID NO: 513, and the sense strand comprises SEQ ID NO: 514;
the antisense strand comprises SEQ ID NO: 515, and the sense strand comprises SEQ ID NO: 516;

the antisense strand comprises SEQ ID NO: 517, and the sense strand comprises SEQ ID NO: 518;
the antisense strand comprises SEQ ID NO: 519, and the sense strand comprises SEQ ID NO: 520;
the antisense strand comprises SEQ ID NO: 521, and the sense strand comprises SEQ ID NO: 522;
the antisense strand comprises SEQ ID NO: 523, and the sense strand comprises SEQ ID NO: 524;
the antisense strand comprises SEQ ID NO: 525, and the sense strand comprises SEQ ID NO: 526; or
the antisense strand comprises SEQ ID NO: 527, and the sense strand comprises SEQ ID NO: 528.

In some embodiments, the siRNA further comprises a targeting group.

In some embodiments, the targeting group comprises an asialoglycoprotein receptor ligand.

In some embodiments, the asialoglycoprotein receptor ligand comprises a galactose cluster.

In some embodiments, the galactose cluster is a monovalent, divalent, trivalent, or tetravalent galactose derivative.

In some embodiments, the galactose derivative is N-acetylgalactosamine (GalNAc).

In some embodiments, the galactose cluster is a trivalent N-acetylgalactosamine.

In some embodiments, the galactose cluster is linked to a branching center via a linker, and the branching center is conjugated to a nucleotide of the siRNA via a linker.

In some embodiments, the targeting group is conjugated to the 5' end of the sense strand.

In some embodiments, the targeting group is conjugated to the 3' end of the sense strand.

In some embodiments, a fragment comprising the targeting group as follows is conjugated to the 5' end or the 3' end of the sense strand (i.e., the following targeting group replaces the hydroxyl group at the 5' end or the 3' end):

HO
OH
OH
AcHN
O
S=P
OH
SH
AcHN
OH
OHO
S

HO
OH
OH
AcHN
O
S=P-OH
S=P-S-
S=P-SH
OH
S-
SH

,

or

In some embodiments, the fragment comprising the targeting group is conjugated to the 5' end of the sense strand.

In some embodiments, the fragment comprising the targeting group is conjugated to the 3' end of the sense strand.

In some embodiments, the sense strand is any one of the sense strands in Table 4.

In some embodiments, the antisense strand is any one of the antisense strands in Table 4.

In some embodiments, the siRNA is any one of the siRNAs in Table 4.

**Table 4**

| siRNA name | Sense strand (SS) and antisense strand (AS) | Sequence | SEQ ID NO: |
|---|---|---|---|
| JAB-190024-1 | antisense strand (3'-5') | dT*dT*mCmGmUfCmGfAmGmGmAmAmUfAmAfCmA mAmU*fA*mU | 529 |
| | sense strand (5'-3') | mG*mC*mAmGmCmUfCfCfUmUmAmUmUmGmUmU mAmU*mA*L96 | 530 |
| JAB-190024-2 | antisense strand (3'-5') | dT*dT*mCmGmUfCmGfAmGmGmAmAmUfAmAfCmA mAmU*fA*mUVP | 531 |
| | sense strand (5'-3') | mG*mC*mAmGmCmUfCfCfUmUmAmUmUmGmUmU mAmU*mA*L96 | 532 |
| JAB-190024-3 | antisense strand (3'-5') | mG*mU*mCfUmAfCmGfAmCfUmCmUmAmAmGfCmG fGmG*fA*mA | 533 |
| | sense strand (5'-3') | mC*mA*mGmAmUmGmCmUfGfAfGmAmUmUmCmG mCmCmCmU*mU*L96 | 534 |
| JAB-190024-4 | antisense strand (3'-5') | mG*mU*mCfUmAfCmGfAmCfUmCmUmAmAmGfCmG fGmG*fA*mAVP | 535 |
| | sense strand (5'-3') | mC*mA*mGmAmUmGmCmUfGfAfGmAmUmUmCmG mCmCmCmU*mU*L96 | 536 |

(continued)

| siRNA name | Sense strand (SS) and antisense strand (AS) | Sequence | SEQ ID NO: |
|---|---|---|---|
| JAB-190024-5 | antisense strand (3'-5') | mU*mG*mUfCmUfCmAfAmUfAmGmCmUmCmCfGmA fGmU*fA*mA | 537 |
| | sense strand (5'-3') | mA*mC*mAmGmAmGmUmUfAfUfCmGmAmGmGmC mUmCmAmU*mU*L96 | 538 |
| JAB-190024-6 | antisense strand (3'-5') | mU*mG*mUfCmUfCmAfAmUfAmGmCmUmCmCfGmA fGmU*fA*mAVP | 539 |
| | sense strand (5'-3') | mA*mC*mAmGmAmGmUmUfAfUfCmGmAmGmGmC mUmCmAmU*mU*L96 | 540 |
| Reference sequence 1 | sense strand (5'-3') | mG*mC*mAmGmCmUfCmCfUfUfAmUmUmGmUmUm AmUmA*dT*dT | 547 |
| | antisense strand (5'-3') | PmU*fA*mUfAmAfCmAfAmUfAmAfGmGfAmGfCmUf GmC*dT*dT | 548 |
| Reference sequence 2 | sense strand (5'-3') | mU*mG*mGmCmAmGfCmUfCfCfUmUmAmUmUmGm UmUmAmUmA | 549 |
| | antisense strand (5'-3') | mU*fA*mUmAmAfCmAmAmUmAmAmGmGfAmGfCm UmGmCmCmA*mC*mA | 550 |
| NAG25-AMG890 | sense strand (5'-3') | NAG25*mC*mAmGmCmCmCmCmUfUfAfUmUmGmU mUmAmUmAmCmG*invdA | 551 |
| | antisense strand (5'-3') | mU*fC*mGfUmAfUmAmAmCmAmAfUmAfAmGfGmGf GmC*fU*mG | 552 |
| GalNAC-SLN360 | sense strand (5'-3') | [ST23*]3/C6XLT*mCmGmGmUmAmAfUfGfGmAmCm AmGmAmGmUmU*mA*mU | 553 |
| | antisense strand (5'-3') | mA*fU*mAfAmCfUmCfUmGfUmCfCmAfUmUfAmC*f C*mG | 554 |

In Table 4, fA, fC, fU, and fG represent 2'-fluoro modified A, C, U, and G ribonucleoside-3'-phosphates, respectively; mA, mC, mU, and mG represent 2'-O-methyl modified A, C, U, and G ribonucleoside-3'-phosphates, respectively; * represents a phosphorothioate linkage; if no * appears between two nucleotides, it indicates a normal phosphodiester linkage between the two nucleotides; dT represents thymidine deoxyribonucleoside-3'-phosphate; P indicates that the nucleotide immediately adjacent to the right side of this P is a 5' phosphate-modified nucleotide; invdA represents an inverted deoxyribonucleotide (3'-3' linked nucleotide); NAG25-AMG890 is olpasiran, and GalNAC-SLN360 is zerlasiran, wherein NAG25 and [ST23*]3/C6XLT have corresponding structures; Reference sequence 1 is modified using the method disclosed in CN117327698A, Reference sequence 2 is modified using the method disclosed in CN116801886A, NAG25-AMG890 is modified using the method disclosed in US9932586B, and GalNAC-SLN360 is modified using the method disclosed in US11499153B. *L96 represents:

.

In some embodiments, each hydroxyl group or thiol group on the phosphodiester linkage or the phosphorothioate linkage in the siRNA is present as a Na salt. (Namely,

and

).

In another aspect, the present application provides a pharmaceutical composition, comprising the siRNA of the present application or a pharmaceutically acceptable salt thereof, and a pharmaceutically acceptable salt.

In another aspect, the present application provides a method of reducing the expression level of LPA in a subject, the method comprising administering to the subject the siRNA of the present application or a pharmaceutically acceptable salt thereof, or the pharmaceutical composition of the present application.

In another aspect, the present application provides use of the siRNA of the present application or a pharmaceutically acceptable salt thereof, or the pharmaceutical composition of the present application in the manufacture of a medicament for preventing or treating a disease associated with LPA expression. In some embodiments, the disease associated with LPA expression is a cardiovascular and cerebrovascular disease. In some embodiments, the cardiovascular and cerebrovascular disease comprises stroke, atherosclerosis, thrombosis, coronary heart disease, or aortic stenosis, and any other disease or pathology associated with elevated levels of Lp(a) particles.

In another aspect, the present application provides a method of preventing or treating a disease associated with LPA expression, the method comprising administering to a subject the siRNA of the present application or a pharmaceutically acceptable salt thereof, or the pharmaceutical composition of the present application. In some embodiments, the disease associated with LPA expression is a cardiovascular and cerebrovascular disease. In some embodiments, the cardiovascular and cerebrovascular disease comprises stroke, atherosclerosis, thrombosis, coronary heart disease, or aortic stenosis, and any other disease or pathology associated with elevated levels of Lp(a) particles.

In another aspect, the present application provides the siRNA of the present application or a pharmaceutically acceptable salt thereof, or the pharmaceutical composition of the present application, for use in preventing or treating a disease associated with LPA expression. In some embodiments, the disease associated with LPA expression is a cardiovascular and cerebrovascular disease. In some embodiments, the cardiovascular and cerebrovascular disease comprises stroke, atherosclerosis, thrombosis, coronary heart disease, or aortic stenosis, and any other disease or pathology associated with elevated levels of Lp(a) particles.

## Definition of Terms

The term "siRNA" (small interfering RNA) refers to a short double-stranded RNA that mediates sequence-specific, effective inhibition of gene expression (which can refer to gene silencing).

The term "modified nucleotide" refers to a nucleotide other than a ribonucleotide (2'-hydroxyl nucleotide). In some embodiments, at least 50%, at least 60%, at least 70%, at least 80%, at least 90%, at least 95%, or 100% of the nucleotides are modified. Modified nucleotides include, but are not limited to, deoxynucleotides, nucleotide mimetics, abasic nucleotides, 2'-modified nucleotides, 3' to 3' linkages (inverted) nucleotides, nucleotides comprising non-natural bases, bridged nucleotides, peptide nucleic acids (PNA), 2',3'-seco nucleotide mimetics (unlocked nucleobase analogs), locked nucleotides, 3'-O-methoxy (2' internucleoside linked) nucleotides, 2'-F-arabinonucleotides, 5'-Me, 2'-fluoro nucleotides, morpholino nucleotides, vinylphosphonate deoxyribonucleotides, vinylphosphonate-containing nucleotides, and cyclopropylphosphonate-containing nucleotides (cPrpN). The 2'-modified nucleotides of the present application (i.e., nucleotides having a group other than a hydroxyl group at the 2' position of the five-membered sugar ring) include, but are not limited to, 2'-O-methyl nucleotides, 2'-deoxy-2'-fluoro nucleotides, 2'-deoxynucleotides, 2'-methoxyethyl (2'-O-2-methoxyethyl) nucleotides, 2'-amino nucleotides, and 2'-alkyl nucleotides. More than one modification can be incorporated into a single LPA siRNA or even into a single nucleotide thereof. LPA siRNA sense strands and antisense strands can be synthesized and/or modified by methods known in the art. The modification at one nucleotide of the present application is independent of the modification at another nucleotide. Modified nucleotides also include nucleotides with modified nucleobases.

Locked nucleic acid (LNA): a class of modified nucleosides in which a methylene bridge is added between C4' and O2' of the ribose.

Glycol nucleic acid (GNA): has a chiral acyclic three-carbon backbone linked by phosphates and is the simplest phosphodiester-based nucleic acid analog.

The term "sequence" or "nucleotide sequence" refers to a series or order of nucleobases, nucleotides, and/or nucleosides. Whether modified or unmodified, it is described by a series of letters using standard nucleotide nomenclature and the symbol table for modified nucleotides described herein.

The term "blunt end" refers to an end of a double-stranded siRNA where the terminal nucleotides of the two annealed strands are complementary (forming a complementary base pair).

The term "bu'xi" refers to an end of a double-stranded siRNA where the terminal nucleotides of the two annealed strands form a pair (i.e., do not form an overhang) but are not complementary (i.e., form a non-complementary pair). In some embodiments, the 5' end of the sense strand and the 3' end of the antisense strand of the siRNA form a frayed end. In some embodiments, the 3' end of the sense strand and the 5' end of the antisense strand of the siRNA form a frayed end. In some embodiments, both ends of the siRNA form frayed ends. In some embodiments, neither end of the siRNA is a frayed end.

The term "overhang" refers to a stretch of one or more unpaired nucleotides at the end of one strand of a double-stranded siRNA. The unpaired nucleotides can be on the sense strand or the antisense strand, resulting in a 3' or 5' overhang. In some embodiments, the siRNA contains: a blunt end and a frayed end, a blunt end and a 5' overhang end, a blunt end and a 3' overhang end, a frayed end and a 5' overhang end, a frayed end and a 3' overhang end, two 5' overhang ends, two 3' overhang ends, a 5' overhang end and a 3' overhang end, two frayed ends, or two blunt ends.

The term "extension" refers to the inclusion of 1, 2, 3, 4, 5, or 6 nucleotides at the 5' and/or 3' ends of the sense strand core sequence and/or the antisense strand core sequence. The extended nucleotides on the sense strand may or may not be complementary to nucleotides (core sequence nucleotides or extended nucleotides) in the corresponding antisense strand. Conversely, the extended nucleotides on the antisense strand may or may not be complementary to nucleotides (core sequence nucleotides or extended nucleotides) in the corresponding sense strand. In some embodiments, both the sense strand and the antisense strand of the siRNA contain 3' and 5' extensions. In some embodiments, the 3' extended nucleotides of one strand base pair with the 5' extended nucleotides of the other strand. In other embodiments, the 3' extended nucleotides of one strand do not base pair with the 5' extended nucleotides of the other strand. In some embodiments, the LPA siRNA has an antisense strand with a 3' extension and a sense strand with a 5' extension.

The term "complementary" when used to describe a first nucleotide sequence (such as an siRNA sense strand or LPA mRNA) with respect to a second nucleotide sequence (such as an siRNA antisense strand) refers to the ability of the nucleotides of the first nucleotide sequence to hybridize (form base pair hydrogen bonds) with the nucleotides of the second nucleotide sequence under certain conditions and form a duplex or double helix structure. Complementary sequences include Watson-Crick base pairs or non-Watson-Crick base pairs, and include natural or modified nucleotides or nucleotide mimetics, as long as the above requirements regarding their ability to hybridize are met.

The term "fully complementary" means that all (100%) bases in a contiguous sequence of a first polynucleotide hybridize with the same number of bases in a contiguous sequence of a second polynucleotide.

The term "partially complementary" means that in a hybridizing nucleobase sequence pair, at least 70% (e.g.,

70%, 75%, 80%, 85%, 90%, 95%, or 100%) of the bases in a contiguous sequence of a first polynucleotide will hybridize with the same number of bases in a contiguous sequence of a second polynucleotide.

The term "substantially complementary" means that in a hybridizing nucleobase sequence pair, at least 85% (e.g., 85%, 90%, 95%, or 100%) of the bases in a contiguous sequence of a first polynucleotide will hybridize with the same number of bases in a contiguous sequence of a second polynucleotide.

The terms "complementary", "fully complementary", and "substantially complementary" can be used with respect to base matching between the sense and antisense strands of an siRNA or between the antisense strand of an siRNA and the sequence of LPA mRNA. Sequence identity or complementarity is independent of modification. For the purpose of determining identity or complementarity, for example, a and Af are complementary to U (or T) and identical to A.

The term "LPA" may refer to the LPA gene, LPA mRNA, or Lp(a) protein, as appropriate.

The terms "silencing", "reducing", "inhibiting", "down-regulating", or "knocking down gene expression", when referring to the LPA gene, mean that when a cell, cell population, or tissue is treated with said LPA siRNA, the expression of the gene (as measured by the level of RNA transcribed from the gene in a cell, cell population, or tissue in which the LPA gene is transcribed, or the level of polypeptide, protein, or protein subunit translated from the mRNA) is reduced compared to the same cell, cell population, or tissue before administration of the LPA siRNA. In some embodiments, the gene expression level and/or mRNA level of LPA in a subject administered said LPA siRNA is reduced by at least about 5%, 10%, 15%, 20%, 25%, 30%, 35%, 40%, 45%, 50%, 55%, 60%, 65%, 70%, 75%, 80%, 85%, 90%, 95%, or 98% relative to the subject before administration of the LPA siRNA or a subject not receiving the LPA siRNA. The gene expression level and/or mRNA level in the subject can be reduced in cells, cell populations, and/or tissues of the subject. In some embodiments, the protein level of LPA in a subject administered said LPA siRNA is reduced by at least about 5%, 10%, 15%, 20%, 25%, 30%, 35%, 40%, 45%, 50%, 55%, 60%, 65%, 70%, 75%, 80%, 85%, 90%, 95%, or 98% relative to the subject before administration of the LPA siRNA or a subject not receiving the LPA siRNA. The protein level in the subject can be reduced in cells, cell populations, tissues, blood, and/or other fluids of the subject. The reduction in gene expression, mRNA, or protein levels can be assessed by any method known in the art. The reduction or decrease in LPA mRNA levels and/or protein levels is collectively referred to herein as reduction or decrease of LPA, or inhibition or reduction of LPA expression.

The term "targeting group" includes, but is not limited to, compounds having affinity for cell surface molecules, cell receptor ligands, haptens, antibodies, monoclonal antibodies, antibody fragments, and antibody mimetics having affinity for somatic cell surface molecules. The targeting group can be monovalent, divalent, trivalent, tetravalent, or have a higher valency. In some embodiments, a linker such as a PEG linker or one, two, or three abasic and/or ribitol groups is used to attach the targeting group to the siRNA. In some embodiments, the targeting group comprises a galactose cluster. The LPA siRNA described herein, having a reactive group (e.g., an amine group) at the 5'-end, can be synthesized. The reactive group can be used subsequently to attach a targeting moiety using methods typical in the art. In some embodiments, the targeting group comprises an asialoglycoprotein receptor ligand. In some embodiments, the asialoglycoprotein receptor ligand comprises or consists of one or more galactose derivatives or a galactose cluster.

The term "galactose cluster" includes molecules having two to four terminal galactose derivatives. The terminal galactose derivatives are attached to the molecule via their C-1 carbon. In some embodiments, the galactose cluster is a galactose derivative trimer, a triantennary galactose derivative, or a trivalent galactose derivative. In some embodiments, the galactose cluster comprises N-acetylgalactosamine (GalNAc). In some embodiments, the galactose cluster comprises trivalent N-acetylgalactosamine. In some embodiments, the galactose cluster is a galactose derivative tetramer, a tetraantennary galactose derivative, or a tetravalent galactose derivative. In some embodiments, the galactose cluster comprises tetravalent N-acetylgalactosamine.

The term "galactose derivative" includes galactose and galactose derivatives having an affinity for the asialoglycoprotein receptor that is equal to or greater than that of galactose. Galactose derivatives include, but are not limited to: galactose, galactosamine, N-formylgalactosamine, N-acetylgalactosamine, N-propionylgalactosamine, N-n-butyrylgalactosamine, and N-isobutyrylgalactosamine. Galactose derivatives and galactose clusters useful for targeting oligonucleotides and other molecules to the liver in vivo are known in the art. Galactose derivatives have been used to target molecules to hepatocytes in vivo by binding to the asialoglycoprotein receptor (ASGPR) expressed on the surface of hepatocytes. Binding of ASGPR ligands to ASGPR promotes cell-specific targeting to hepatocytes and endocytosis of the molecule into hepatocytes. Galactose clusters can be attached to the 3' or 5' end of a polynucleotide of an siRNA using methods known in the art.

The term "galactose derivative trimer" contains three galactose derivatives, each attached to a central branching point. In some embodiments, the galactose cluster consists of a galactose derivative trimer, which is an N-acetylgalactosamine trimer.

The term "galactose derivative tetramer" contains four galactose derivatives, each attached to a central branching point. In some embodiments, the galactose cluster consists of a galactose derivative tetramer, which is an N-acetylgalactosamine tetramer.

The term "linker or spacer" is a group that connects a galactose derivative to a branching point or attaches a branching point to an siRNA.

The term "branching point" refers to any small molecule that allows the attachment of multiple (e.g., three or four) galactose derivatives and further allows the attachment of the branching point to the siRNA.

The term "linking group" refers to a linker or connecting group that facilitates covalent bonding of said LPA siRNA to a targeting group or a delivery polymer or delivery vehicle. The linking group can be attached to the 3' or 5' end of the sense strand or antisense strand of the siRNA. In some embodiments, the linking group is attached to the sense strand of the siRNA. In some embodiments, the linking group is conjugated to the 5' or 3' end of the sense strand of the siRNA. In some embodiments, the linking group is conjugated to the 5' end of the sense strand of the siRNA.

A linker or linking group is a connection between two atoms that connects one target chemical group (such as an siRNA) or segment to another target chemical group (such as a targeting group or delivery polymer) or segment via one or two covalent bonds. A labile linkage contains a labile bond. A linkage may optionally include a spacer that increases the distance between the two connected atoms. A spacer can also add flexibility and/or length to the linkage. Spacers can include, but are not limited to, alkyl groups, alkenyl groups, alkynyl groups, aryl groups, aralkyl groups, arylalkenyl groups, and arylalkynyl groups; each of which may contain one or more heteroatoms, heterocycles, amino acids, nucleotides, and sugars. Spacer groups are well known in the art, and the foregoing list is not intended to limit the scope of this specification.

The term "delivery vehicle" refers to a substance that can be used to deliver an siRNA to a cell or tissue. A delivery vehicle is a compound that improves the delivery of an siRNA to a cell or tissue. In some embodiments, the siRNA can be combined with lipids, nanoparticles, polymers, liposomes, micelles, or other delivery systems available in the art. The siRNA can also be chemically conjugated to targeting groups, lipids (including but not limited to cholesterol and cholesteryl derivatives), nanoparticles, polymers, liposomes, micelles, or other delivery systems available in the art. In some embodiments, the LPA siRNA is linked to a targeting ligand comprising an asialoglycoprotein ligand. In some embodiments, the LPA siRNA is linked to a targeting ligand comprising or consisting of a galactose cluster.

The term "pharmaceutical composition" includes a pharmacologically effective amount of at least one LPA siRNA of the present application and one or more pharmaceutically acceptable excipients. A pharmaceutically acceptable excipient is a substance other than the active pharmaceutical ingredient (API, therapeutic product, such as LPA siRNA) that has been appropriately safety evaluated and is intentionally included in a drug delivery system. Excipients do not exert or are not intended to exert a therapeutic effect at the intended dosage. The siRNA and the pharmaceutical composition comprising the LPA siRNA disclosed herein can be packaged or included in a kit, container, package, or dispenser. The LPA siRNA and the pharmaceutical composition comprising the LPA siRNA can be packaged in a pre-filled syringe or vial. These pharmaceutical compositions can be used to inhibit the expression of the LPA gene in a cell, tissue, or organism. In some embodiments, the pharmaceutical composition is used to treat a subject suffering from a disease, disorder, or condition that would benefit from reduced or inhibited LPA expression. In some embodiments, the pharmaceutical composition is used to treat a subject at risk of developing a disease, disorder, or condition that would benefit from reduced or inhibited LPA expression. Diseases, disorders, or conditions that would benefit from reduced or inhibited LPA expression include, but are not limited to: Buerger's disease, peripheral artery disease, coronary artery disease, metabolic syndrome, acute coronary syndrome, aortic stenosis, aortic regurgitation, aortic dissection, retinal artery obstruction, cerebrovascular disease, mesenteric ischemia, superior mesenteric artery obstruction, renal artery stenosis, stable/-unstable angina pectoris, acute coronary syndrome, heterozygous or homozygous familial hypercholesterolemia, hyperapobetalipoproteinemia, cerebrovascular atherosclerosis, cerebrovascular disease, and venous thrombosis. In some embodiments, the subject is a mammal, including but not limited to a human patient.

Delivery of the siRNA to cells, tissues, human organisms, or non-human organisms is achieved by any means available in the art. In some embodiments, the cells are mammalian cells, including but not limited to human cells. The cells, tissues, or non-human organisms can be used for research or as research tools (e.g., drug testing or diagnostics).

The LPA siRNA described herein can be used to treat a subject suffering from or at risk of suffering from a disease, disorder, or condition that would benefit from reduced or inhibited LPA expression. Treatment of a subject that would benefit from reduced and/or inhibited LPA gene expression includes therapeutic and/or prophylactic treatment. Examples of diseases, disorders, or conditions include, but are not limited to: Buerger's disease, peripheral artery disease, coronary artery disease, metabolic syndrome, acute coronary syndrome, aortic stenosis, aortic regurgitation, aortic dissection, retinal artery obstruction, cerebrovascular disease, mesenteric ischemia, superior mesenteric artery obstruction, renal artery stenosis, stable/unstable angina pectoris, acute coronary syndrome, heterozygous or homozygous familial hypercholesterolemia, hyperapobetalipoproteinemia, cerebrovascular atherosclerosis, cerebrovascular disease, and venous thrombosis. In some embodiments, the method includes administering a composition, such as a pharmaceutical composition comprising an LPA siRNA described herein, to the mammal to be treated.

In some embodiments, a therapeutically effective amount of one or more of said LPA siRNAs is administered to a subject, thereby inhibiting LPA expression in the subject (e.g., an amount effective to inhibit LPA expression in the subject). In some embodiments, one or more LPA siRNAs described herein are used to treat a subject suffering from a disease or condition that would benefit from reduced or inhibited LPA expression. In some embodiments, the LPA siRNA is used to treat or prevent at least one symptom in a subject suffering from a disease or condition that would benefit from reduced or inhibited LPA expression. A therapeutically effective amount of any one or more of said siRNAs is administered to the

subject, thereby treating the symptom. In some embodiments, a prophylactically effective amount of any one or more of said siRNAs is administered to the subject, thereby preventing at least one symptom.

In some embodiments, an LPA siRNA is used to treat or manage a clinical presentation, wherein a therapeutically or prophylactically effective amount of one or more LPA siRNAs or a composition comprising an LPA siRNA described herein is administered to a subject in need of such treatment, prevention, or management. In some embodiments, the method includes administering a composition comprising an LPA siRNA described herein to the mammal to be treated.

The route of administration is the route by which the LPA siRNA contacts the body. Generally, methods of administering pharmaceuticals and nucleic acids for treating subjects are well known in the art and can be applied to the administration of the compositions described herein. The compounds described herein can be administered via any suitable route in a formulation appropriately tailored for the specific route. Thus, the compounds described herein can be administered by injection, e.g., intravenously, intramuscularly, intradermally, subcutaneously, or intraperitoneally.

## DESCRIPTION OF THE DRAWINGS

The present application can better understand the features and advantages involved in the present application by referring to the exemplary embodiments described in detail below and the drawings. A brief description of the drawings is as follows:

Figure 1 is a flow diagram of the solid-phase phosphoramidite technology for synthesizing the LPA siRNA agent of the present application.
Figure 2 is a graph showing the effect of the RNA inhibitors in Table 7 prepared in Example 4 of the present application at different concentrations on reducing the level of Lp(a) in the serum of transgenic mice.
Figure 3 is a graph showing the effect of the RNA inhibitors in Table 8 prepared in Example 5 of the present application at different concentrations on reducing the level of Lp(a) in the serum of transgenic mice.
Figure 4 is a graph showing the effect of the RNA inhibitors in Table 9 prepared in Example 6 of the present application on reducing the level of Lp(a) in the serum of cynomolgus monkeys.

## DETAILED DESCRIPTION

Hereinafter, the present application is further described with reference to the examples.

### Example 1: Design and Synthesis of LPA siRNA

For the lipoprotein gene (transcript information: NM005577.4), small interfering nucleic acids (siRNAs), as shown in Table 1, were designed in the present application. The transcript information of this protein gene is recorded in the NCBI database. In Table 1, the names indicate the sequences of siRNAs targeting LPA mRNA, as well as their sense strand and antisense strand sequences. Table 2 shows siRNAs, their sense strands, and antisense strands obtained by adding two dTs to the 3' ends of the sense strands and antisense strands based on Table 1. Subsequently, the above sequences were modified in the present application, and the modified siRNAs, the sense strands, and the antisense strands thereof are shown in Table 3. The siRNAs with targeting group modifications, the sense strands, and the antisense strands thereof are shown in Table 4. The siRNAs disclosed in the prior art, as well as the sense strands and antisense strands thereof, are also shown in Table 4.

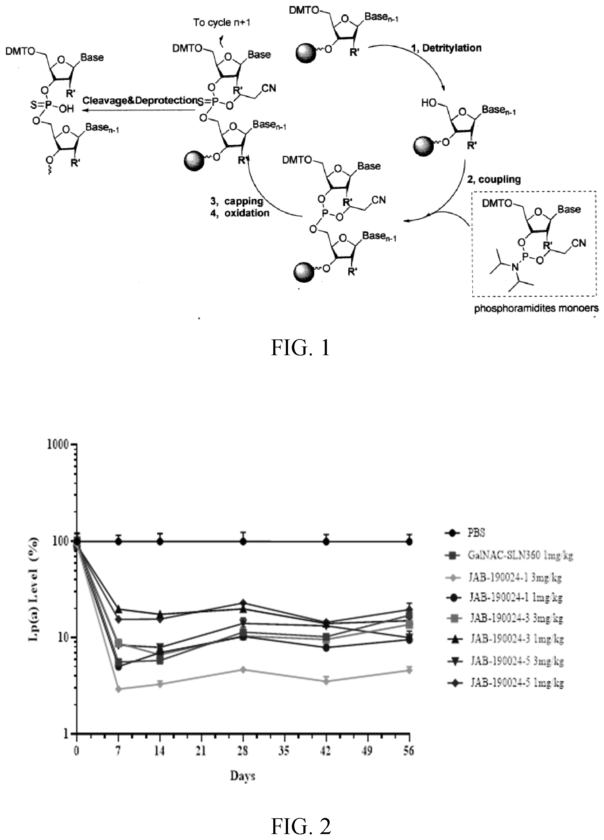

The LPA siRNA agent of the present application can be synthesized using conventional solid-phase phosphoramidite technology in the art, as shown in Figure 1. All materials are commercially available. Briefly, first, the DMT protecting group was removed using TCA; then, washing was performed using ACN; then, coupling, capping, oxidation/sulfurization, and ACN washing were performed, followed by repeating the coupling cycle. After the target sequence was synthesized, aminolysis was performed using aqueous ammonia at 40°C for 16 hours, followed by centrifugal drying. Then, the product was obtained by desalting or HPLC purification. For UPLC/TOF-MS detection and UV concentration quantification, 4 μL of the sample was added to 196 μL of water in a UV plate; the concentration was detected using a microplate reader, and the concentration in nmol was calculated. Annealing: the antisense and sense strands were mixed and annealed in a molar ratio of 1.05:1, aliquoted into 2.5 nmol × 2 portions (in appropriate centrifuge tubes), and then lyophilized in individual tubes, ensuring a small amount of solution, and the tubes were sealed with aluminum foil during lyophilization to obtain the final siRNA.

### Example 2: Detection of LPA Expression Level

The expression level of LPA was evaluated using a dual-luciferase reporter gene assay. First, a psiCHECK2-LPA dual-luciferase reporter gene plasmid was constructed. The full-length LPA sequence was inserted into the psiCHECK2-

LPA vector. The LPA sequence was obtained from Origene, and the psiCHECK2 vector was obtained from Promega. HUH7 cells were used as the main experimental material. The cells were seeded in 96-well plates and cultured in a 37°C incubator for 2 hours to allow the cells to adhere. Then, the LPA dual-reporter gene plasmid was transfected into HUH7 cells using FugeneHD, and the cells were cultured overnight in a 5% $CO_2$, 37°C incubator. After overnight culture for 16 hours, LPA-siRNA was transfected into the HUH7 cells using RNAiMAX, and the cells were cultured for 24 hours. RNAiMax was obtained from Invitrogen. After culturing the HUH7 cells in a 5% $CO_2$, 37°C incubator for 24 hours, the expression of Firefly luciferase and Renilla luciferase was detected using the Dual-Glo Luciferase Assay System. The calculation formulas for the absolute inhibition rate and relative inhibition rate of LPA are as follows, where "sample" represents the group added with 0.3 nM of the siRNA to be tested, "internal control" represents the group without added siRNA, and "control" represents the group added with 0.3 nM of control siRNA.

LPA inhibition rate (%) = { 1 - [(Renilla lum. of sample - average of background Renilla lum.) / (Firefly lum. of sample - average of background Firefly lum.)] / [(Renilla lum. of internal control - average of background Renilla lum.) / (Firefly lum. of internal control - average of background Firefly lum.)] } × 100%. The LPA inhibition rate of the control siRNA was 60.38%.

LPA relative inhibition rate (%) = (LPA inhibition rate of sample / LPA inhibition rate of control) × 100%.

| Name | Sense strand (5'-3') | Antisense strand (3'-5') |
|---|---|---|
| Control siRNA | CAGCCCCUUAUUGUUAUACGA (SEQ ID NO: 555) | GUCGGGGAAUAACAAUAUGCU (SEQ ID NO: 556) |

The obtained results are shown in **Table 5** below:

**Table 5**

| siRNA name | Relative inhibition rate | siRNA name | Relative inhibition rate |
|---|---|---|---|
| 190024001dt | 120% | 190024064dt | 48% |
| 190024002dt | 114% | 190024065dt | 48% |
| 190024003dt | 113% | 190024066dt | 47% |
| 190024004dt | 112% | 190024067dt | 47% |
| 190024005dt | 110% | 190024068dt | 47% |
| 190024006dt | 108% | 190024069dt | 47% |
| 190024007dt | 108% | 190024070dt | 46% |
| 190024008dt | 107% | 190024071dt | 45% |
| 190024009dt | 106% | 190024072dt | 45% |
| 190024010dt | 103% | 190024073dt | 45% |
| 190024011dt | 102% | 190024074dt | 44% |
| 190024012dt | 100% | 190024075dt | 42% |
| 190024013dt | 98% | 190024076dt | 42% |
| 190024014dt | 97% | 190024077dt | 41% |
| 190024015dt | 97% | 190024078dt | 41% |
| 190024016dt | 95% | 190024079dt | 39% |
| 190024017dt | 93% | 190024080dt | 39% |
| 190024018dt | 88% | 190024081dt | 39% |
| 190024019dt | 86% | 190024082dt | 39% |
| 190024020dt | 86% | 190024083dt | 38% |
| 190024021dt | 83% | 190024084dt | 38% |

(continued)

| siRNA name | Relative inhibition rate | siRNA name | Relative inhibition rate |
|---|---|---|---|
| 190024022dt | 82% | 190024085dt | 38% |
| 190024023dt | 81% | 190024086dt | 37% |
| 190024024dt | 79% | 190024087dt | 37% |
| 190024025dt | 78% | 190024088dt | 37% |
| 190024026dt | 77% | 190024089dt | 36% |
| 190024027dt | 76% | 190024090dt | 34% |
| 190024028dt | 75% | 190024091dt | 33% |
| 190024029dt | 75% | 190024092dt | 33% |
| 190024030dt | 74% | 190024093dt | 33% |
| 190024031dt | 74% | 190024094dt | 33% |
| 190024032dt | 73% | 190024095dt | 33% |
| 190024033dt | 73% | 190024096dt | 32% |
| 190024034dt | 72% | 190024097dt | 31% |
| 190024035dt | 72% | 190024098dt | 31% |
| 190024036dt | 72% | 190024099dt | 30% |
| 190024037dt | 72% | 190024100dt | 28% |
| 190024038dt | 71% | 190024101dt | 28% |
| 190024039dt | 69% | 190024102dt | 27% |
| 190024040dt | 69% | 190024103dt | 26% |
| 190024041dt | 67% | 190024104dt | 25% |
| 190024042dt | 64% | 190024105dt | 24% |
| 190024043dt | 62% | 190024106dt | 24% |
| 190024044dt | 61% | 190024107dt | 23% |
| 190024045dt | 60% | 190024108dt | 23% |
| 190024046dt | 60% | 190024109dt | 23% |
| 190024047dt | 60% | 190024110dt | 22% |
| 190024048dt | 59% | 190024111dt | 20% |
| 190024049dt | 59% | 190024112dt | 19% |
| 190024050dt | 59% | 190024113dt | 18% |
| 190024051dt | 58% | 190024114dt | 17% |
| 190024052dt | 57% | 190024115dt | 16% |
| 190024053dt | 56% | 190024116dt | 14% |
| 190024054dt | 55% | 190024117dt | 14% |
| 190024055dt | 54% | 190024118dt | 14% |
| 190024056dt | 52% | 190024119dt | 14% |
| 190024057dt | 52% | 190024120dt | 14% |
| 190024058dt | 51% | 190024121dt | 13% |
| 190024059dt | 51% | 190024122dt | 13% |
| 190024060dt | 50% | 190024123dt | 7% |

(continued)

| siRNA name | Relative inhibition rate | siRNA name | Relative inhibition rate |
|---|---|---|---|
| 190024061dt | 49% | 190024124dt | 1% |
| 190024062dt | 49% | 190024125dt | 0% |
| 190024063dt | 49% | | |

**Example 3: In Vitro Experiment of LPA RNAi Agent**

The expression level of LPA was evaluated using a dual-luciferase reporter gene assay. First, a psiCHECK2-LPA dual-luciferase reporter gene plasmid was constructed. The full-length human LPA sequence was inserted into the psiCHECK2 vector. The LPA sequence was obtained from Origene, and the psiCHECK2 vector was obtained from Promega. Hep3B cells were seeded at 10,000 cells per well in a 96-well plate and cultured overnight in a 37°C incubator to allow the cells to adhere. The LPA reporter gene plasmid was transfected into the cells using 0.2 microliters per well of FUGENEHD (Promega, E2311). After culturing the cells in the incubator for 6 hours, the siRNA was transfected into the cells using 0.2 microliters per well of lipofectamine 2000 (Thermo, 11668-019), and the cells were cultured in a 5% $CO_2$, 37°C incubator for 24 hours. The expression of Firefly luciferase and Renilla luciferase was detected using the Dual-Glo Luciferase Assay System (Promega E29440) to determine the expression level of LPA.

LPA inhibition rate (%) = {1 - [(Renilla lum. of sample - average of background Renilla lum.) / (Firefly lum. of sample - average of background Firefly lum.)] / [(Renilla lum. of internal control - average of background Renilla lum.) / (Firefly lum. of internal control - average of background Firefly lum.)]} × 100%. The $IC_{50}$ value was calculated and analyzed using the dose-response (variable slope) four-parameter method in GraphPad Prism software. The obtained results are shown in **Table 6** below:

**Table 6**

| Compd ID | $IC_{50}$ (nM) | Top inhibition (%) |
|---|---|---|
| 190024-M1 | 0.77 | 54.88 |
| 190024-M8 | 0.41 | 78.01 |
| 190024-M2 | 0.23 | 83.84 |
| 190024-M5 | 0.10 | 85.84 |
| 190024-M3 | 0.25 | 71.19 |
| 190024-M4 | 0.14 | 79.35 |
| 190024-M7 | 0.40 | 78.04 |
| 190024-M9 | 0.50 | 78.61 |
| 190024-M12 | 0.31 | 84.45 |
| 190024-M10 | 0.86 | 65.72 |
| 190024-M11 | 0.47 | 75.66 |
| NAG25-AMG890 | 0.41 | 79.97 |
| Reference sequence 1 | 0.45 | 69.75 |
| Reference sequence 2 | >2.5 | 33.08 |

**Example 4: In Vivo Detection of Efficacy of LPA RNAi Agent in Transgenic Mice**

To evaluate the in vivo activity of the siRNAs to be tested, LPA humanized transgenic mice were used in this experiment to evaluate the in vivo activity of the molecules to be tested. On day 0, transgenic mice were subcutaneously injected with 3 mg/kg of JAB-190024-1, JAB-190024-3, JAB-190024-5, and 1 mg/kg of JAB-190024-1, JAB-190024-3, JAB-190024-5, and SLN360. Mice in the control group were injected with an equal volume of phosphate buffered saline (PBS). Mouse serum was collected before administration and on days 7, 14, 28, 42, and 56 after administration. The level of Lp(a) in mouse serum was detected using the Human Lipoprotein(a) ELISA (Abcam ab212165) kit. Normalization was performed based on the Lp(a) level in serum samples from the PBS-treated group to calculate the relative Lp(a) level in the

administration groups. The obtained results are shown in **Table 7** and **Figure 2**:

**Table 7**

| | Day 0 | Day 7 | Day 14 | Day 28 | Day 42 | Day 56 |
|---|---|---|---|---|---|---|
| | Mean ± SD (%) | Mean ± SD (%) | Mean ± SD (%) | Mean ± SD (%) | Mean ± SD (%) | Mean ± SD (%) |
| **PBS** | 100±50.4 | 100±35.3 | 100±46.5 | 100±54.8 | 100±41 | 100±41 |
| GalNAC-SLN360 1mg/kg | 103.7±35.8 | 5.6±1.0 | 5.8±1.4 | 11.5±3.3 | 10.2±0.8 | 17.1±0.7 |
| JAB-190024-1 3mg/kg | 93.3±25.5 | 2.9±0.4 | 3.3±0.6 | 4.7±0.6 | 3.5±0.9 | 4.6±0.9 |
| JAB-190024-1 1mg/kg | 90.4±26.9 | 5.0±1.2 | 7.0±1.5 | 10.3±3.0 | 7.9±1.4 | 9.6±1.2 |
| JAB-190024-3 3mg/kg | 96.0±26.5 | 8.8±1.9 | 6.7±1.4 | 10.4±2.7 | 9.6±2.9 | 13.8±2.7 |
| JAB-190024-3 1mg/kg | 91.7±29.4 | 19.9±3.2 | 17.5±3.1 | 20.0±6.8 | 14.1±2.6 | 15.1±3.1 |
| JAB-190024-5 3mg/kg | 93.1±27.0 | 8.4±2.2 | 8.0±0.9 | 14.1±4.3 | 13.3±3.6 | 10.1±3.7 |
| JAB-190024-5 1mg/kg | 90.3±24.3 | 15.6±2.7 | 15.7±2.7 | 23.0±2.7 | 14.4±2.1 | 19.7±7.1 |

**Example 5: In Vivo Detection of Efficacy of LPA RNAi Agent in Transgenic Mice**

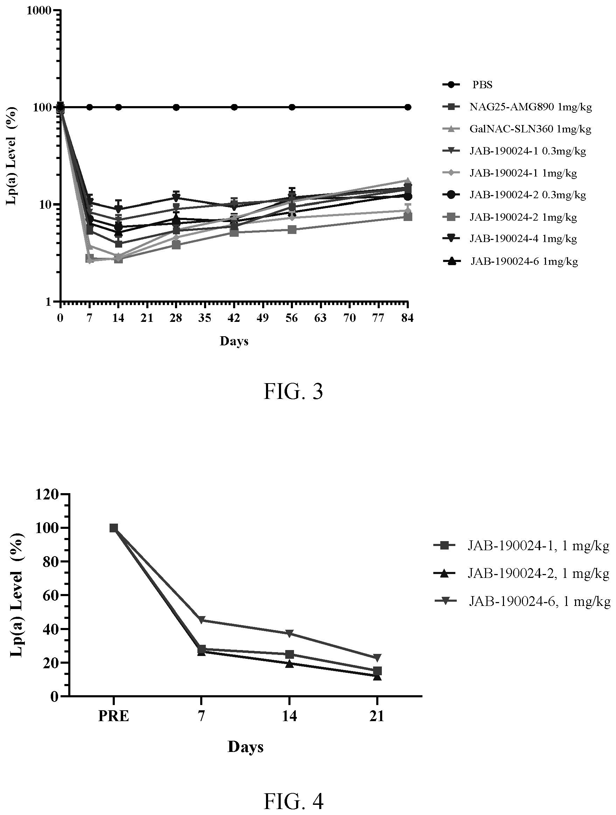

To evaluate the in vivo activity of the siRNAs to be tested, LPA humanized transgenic mice were used in this experiment to evaluate the in vivo activity of the molecules to be tested. On day 0, transgenic mice were subcutaneously injected with 1 mg/kg of AMG890, SLN360, JAB-190024-1, JAB-190024-2, JAB-190024-6, and JAB-190024-4, and 0.3 mg/kg of JAB-190024-1, JAB-190024-2, JAB-190024-6, and JAB-190024-4. Mice in the control group were injected with an equal volume of phosphate buffered saline (PBS). Mouse serum was collected on the day before administration (day -1) and on days 7, 14, 28, 42, 56, and 84 after administration. The level of human Lp(a) in mouse serum was detected using Human Lipoprotein(a) ELISA (Abcam, ab212165). Normalization was performed based on the Lp(a) level in serum samples from the PBS-treated group to calculate the relative Lp(a) level in the administration groups. The obtained results are shown in **Table 8** and **Figure 3**:

**Table 8**

| | Day 0 | Day 7 | Day 14 | Day 28 | Day 42 | Day 56 | Day 84 |
|---|---|---|---|---|---|---|---|
| | Mean ± SD (%) | Mean ± SD (%) | Mean ± SD (%) | Mean ± SD (%) | Mean ± SD (%) | Mean ± SD (%) | Mean ± SD (%) |
| **PBS** | 100±24.8 | 100±11.0 | 100±16.3 | 100±9.5 | 100±16.6 | 100±8.8 | 100±5.4 |
| NAG25-AMG890 1mg/kg | 98.9±23.1 | 5.3±1.4 | 3.9±1.6 | 5.4±1.5 | 5.9±1.6 | 9.5±3.8 | 14.3±3.7 |
| GalNAC-SLN360 1mg/kg | 100.8±25.3 | 3.7±0.5 | 3.0±0.4 | 5.4±0.4 | 7.3±0.7 | 10.6±1.6 | 17.6±2.1 |
| JAB-190024-1 0.3mg/kg | 98.5±16.5 | 8.4±3.0 | 6.9±1.9 | 9.0±2.2 | 10.2±3.0 | 11.0±4.0 | 14.5±4.9 |
| JAB-190024-1 1mg/kg | 98.1±21.2 | 2.6±0.3 | 2.8±0.6 | 4.6±1.3 | 6.2±3.4 | 7.3±2.4 | 8.7±2.9 |
| JAB-190024-2 0.3mg/kg | 97.8±18.5 | 7.1±2.3 | 5.9±1.4 | 6.4±0.8 | 7.1±2.0 | 11.3±7.6 | 12.0±2.7 |
| JAB-190024-2 1mg/kg | 97.3±23.9 | 2.8±0.5 | 2.7±0.5 | 3.8±0.9 | 5.1±1.4 | 5.5±1.0 | 7.5±1.9 |
| JAB-190024-6 1mg/kg | 94.2±12.7 | 6.4±1.7 | 5.2±2.1 | 7.2±2.6 | 6.7±2.1 | 8.3±2.3 | 12.6±1.8 |
| JAB-190024-4 1mg/kg | 94.9±15.5 | 10.4±4.6 | 8.9±4.6 | 11.6±4.3 | 9.4±2.7 | 11.7±3.6 | 14.8±3.7 |

**Example 6: In Vivo Detection of Efficacy of LPA RNAi Agent in Cynomolgus Monkeys**

To evaluate the in vivo activity of the siRNAs to be tested in large animals, cynomolgus monkeys (male, aged 4-7 years, weighing 3-6 kg) were used in this experiment to evaluate the in vivo activity of the molecules to be tested. The cynomolgus monkeys were housed in an animal room with environmental conditions controlled at a temperature of 18°C to 26°C and a relative humidity of 40% to 70%. The cynomolgus monkeys were subcutaneously injected with 1 mg/kg of JAB-190024-1, JAB-190024-2, and JAB-190024-6. Serum was collected before administration and on days 7, 14, and 21 after administration. The collected 0.5 mL of serum was aliquoted into two Eppendorf tubes and stored in a -80°C freezer for subsequent Lp(a) level detection. The Lp(a) level in the serum was detected using the Mercodia Lp(a) Elisa (Mercodia, 10-1106-01) kit. Normalization was performed based on the serum Lp(a) level of the cynomolgus monkeys before administration to calculate the relative Lp(a) level of each group at different time points after administration. The obtained results are shown in **Table 9** and **Figure 4**.

**Table 9**

| | **Day 7, Lp(a) expression% relative to pre-administration** | **Day 14, Lp(a) expression% relative to pre-administration** | **Day 21, Lp(a) expression% relative to pre-administration** |
|---|---|---|---|
| | **Mean** | **Mean** | **Mean** |
| JAB-190024-1 1mg/kg | 28.3 | 25.1 | 15.4 |
| JAB-190024-2 1mg/kg | 26.8 | 19.6 | 12.2 |
| JAB-190024-6 1mg/kg | 45.3 | 37.3 | 22.8 |

**Example 7**

The experiment for nucleic acid-protein interaction was performed based on the surface plasmon resonance principle using a GE Biacore T200 instrument and a Cytiva S series SA chip.

According to the manufacturer's manual, using the standard ligand nucleic acid coupling procedure, the biotinylated modified AS strand RNA ligand was coupled to the surface of a Sensor Chip SA chip (Cytiva, Catalog No. BR-1005-31) channel, with the target coupling amount set to 500 RU.

For kinetic measurements, at 25°C in 1X HBS-EP+ buffer, using a contact time of 180 seconds and a flow rate of 30 μL/min, Recombinant Human Argonaute-2/AGO2 Protein (His Tag) (SinoBiological, Catalog No. 11079-H07B) was applied at increasing concentrations, and the signal was monitored over time. Specifically, the sample solution was injected onto the chip surface at a flow rate of 30 μL/min for 180 seconds. After each binding reaction, dissociation was allowed to occur for 1200 seconds. 0.5% SDS as a regeneration reagent was injected at a flow rate of 30 μL/min for 30 seconds to return the signal to baseline. The 1:1 binding model was used to fit the data. The sequences tested and the results obtained are shown in **Table 10** and **Table 11** below, respectively.

**Table 10**

| RNA | 5'-3' |
|---|---|
| JAB-1900242AS-biotin (SEQ ID NO: 557) | VpmU*fA*mUmAmAfCmAfAmUmAmAmGmGfAmGfCmUmGmC*dT*dT(biotin) |
| Reference sequence 1-AS-biotin (SEQ ID NO: 558) | PmU*fA*mUfAmAfCmAfAmUfAmAfGmGfAmGfCmUfGmC*dT*dT(biotin) |
| Reference sequence 2-AS-biotin (SEQ ID NO: 559) | mU*fA*mUmAmAfCmAmAmUmAmAmGmGfAmGfCmUmGmCmCmA*mC*mA( biotin) |
| JAB-1900241AS-biotin (SEQ ID NO: 560) | mU*fA*mUmAmAfCmAfAmUmAmAmGmGfAmGfCmUmGmC*dT*dT(biotin) |

**Table 11**

| RNA | KD (M) |
|---|---|
| JAB-1900242AS-biotin | 8.556E-11 |
| Reference sequence 1-AS-biotin | 6.370E-10 |
| Reference sequence 2-AS-biotin | 5.527E-10 |
| JAB-1900241AS-biotin | 3.644E-10 |

Although the embodiments of the present application have been shown and described above, it is to be understood that the above embodiments are exemplary and are not to be construed as limiting the present application. A person of ordinary skill in the art may make changes, modifications, substitutions, and variations to the above embodiments within the scope of the present application.

## Claims

1. An siRNA or a pharmaceutically acceptable salt thereof, comprising a sense strand and an antisense strand, wherein the antisense strand comprises a sequence complementary to an LPA mRNA sequence, and a sequence of the antisense strand comprises any one of the sequences of the antisense strands in Table 1.

2. The siRNA according to claim 1, or the pharmaceutically acceptable salt thereof, wherein the antisense strand is 19 to 26 nucleobases in length.

3. The siRNA according to claim 2, or the pharmaceutically acceptable salt thereof, wherein the antisense strand is 19 to 21 nucleobases in length.

4. The siRNA according to any one of claims 1-3, or the pharmaceutically acceptable salt thereof, wherein the sense strand comprises a sequence substantially complementary to the antisense strand, and forms a duplex complementary region of 15 to 26 contiguous base pairs.

5. The siRNA according to claim 4, or the pharmaceutically acceptable salt thereof, wherein the duplex complementary region is 18 to 23 contiguous base pairs in length.

6. The siRNA according to claim 5, or the pharmaceutically acceptable salt thereof, wherein the duplex complementary region is 19 to 21 contiguous base pairs in length.

7. The siRNA according to any one of claims 1-6, or the pharmaceutically acceptable salt thereof, wherein the sense strand is 19 to 26 nucleobases in length.

8. The siRNA according to any one of claims 1-7, or the pharmaceutically acceptable salt thereof, wherein the sense strand is 19 to 21 nucleobases in length.

9. The siRNA according to any one of claims 1-8, or the pharmaceutically acceptable salt thereof, wherein a sequence of the sense strand comprises any one of the sequences of the sense strands in Table 1.

10. The siRNA according to any one of claims 1-9, or the pharmaceutically acceptable salt thereof, wherein the antisense strand comprises any one of the sequences of SEQ ID NOs: 128-150 and 253-254.

11. The siRNA according to any one of claims 1-10, or the pharmaceutically acceptable salt thereof, wherein the sense strand comprises any one of the sequences of SEQ ID NOs: 1-23 and 126-127.

12. The siRNA according to any one of claims 1-11, or the pharmaceutically acceptable salt thereof, wherein the siRNA comprises any one of the siRNAs in Table 1.

13. The siRNA according to any one of claims 1-12, or the pharmaceutically acceptable salt thereof, wherein in the siRNA:

the antisense strand comprises SEQ ID NO: 128, and the sense strand comprises SEQ ID NO: 1;
the antisense strand comprises SEQ ID NO: 129, and the sense strand comprises SEQ ID NO: 2;
the antisense strand comprises SEQ ID NO: 130, and the sense strand comprises SEQ ID NO: 3;
the antisense strand comprises SEQ ID NO: 131, and the sense strand comprises SEQ ID NO: 4;
the antisense strand comprises SEQ ID NO: 132, and the sense strand comprises SEQ ID NO: 5;
the antisense strand comprises SEQ ID NO: 133, and the sense strand comprises SEQ ID NO: 6;
the antisense strand comprises SEQ ID NO: 134, and the sense strand comprises SEQ ID NO: 7;
the antisense strand comprises SEQ ID NO: 135, and the sense strand comprises SEQ ID NO: 8;
the antisense strand comprises SEQ ID NO: 136, and the sense strand comprises SEQ ID NO: 9;
the antisense strand comprises SEQ ID NO: 137, and the sense strand comprises SEQ ID NO: 10;
the antisense strand comprises SEQ ID NO: 138, and the sense strand comprises SEQ ID NO: 11;
the antisense strand comprises SEQ ID NO: 139, and the sense strand comprises SEQ ID NO: 12;
the antisense strand comprises SEQ ID NO: 140, and the sense strand comprises SEQ ID NO: 13;
the antisense strand comprises SEQ ID NO: 141, and the sense strand comprises SEQ ID NO: 14;
the antisense strand comprises SEQ ID NO: 142, and the sense strand comprises SEQ ID NO: 15;
the antisense strand comprises SEQ ID NO: 143, and the sense strand comprises SEQ ID NO: 16;
the antisense strand comprises SEQ ID NO: 144, and the sense strand comprises SEQ ID NO: 17;
the antisense strand comprises SEQ ID NO: 145, and the sense strand comprises SEQ ID NO: 18;
the antisense strand comprises SEQ ID NO: 146, and the sense strand comprises SEQ ID NO: 19;
the antisense strand comprises SEQ ID NO: 147, and the sense strand comprises SEQ ID NO: 20;
the antisense strand comprises SEQ ID NO: 148, and the sense strand comprises SEQ ID NO: 21;
the antisense strand comprises SEQ ID NO: 149, and the sense strand comprises SEQ ID NO: 22;
the antisense strand comprises SEQ ID NO: 150, and the sense strand comprises SEQ ID NO: 23;
the antisense strand comprises SEQ ID NO: 253, and the sense strand comprises SEQ ID NO: 126; or
the antisense strand comprises SEQ ID NO: 254, and the sense strand comprises SEQ ID NO: 127.

**14.** The siRNA according to any one of claims 1-13, or the pharmaceutically acceptable salt thereof, wherein the antisense strand or the sense strand contains 1, 2, 3, 4, 5, or 6 extensions.

**15.** The siRNA according to any one of claims 1-14, or the pharmaceutically acceptable salt thereof, wherein the extensions of the antisense strand or the sense strand are dTs.

**16.** The siRNA according to any one of claims 1-15, or the pharmaceutically acceptable salt thereof, wherein the antisense strand or the sense strand contains 2 dT extensions.

**17.** The siRNA according to any one of claims 1-15, or the pharmaceutically acceptable salt thereof, wherein the 3' end of the antisense strand contains two extended dTs, and the sense strand does not contain dT.

**18.** The siRNA according to any one of claims 1-17, or the pharmaceutically acceptable salt thereof, wherein the antisense strand comprises any one of the sequences of the antisense strands in Table 2.

**19.** The siRNA according to any one of claims 1-18, or the pharmaceutically acceptable salt thereof, wherein the antisense strand comprises any one of the sequences of SEQ ID NOs: 380-402 and 544-546.

**20.** The siRNA according to any one of claims 1-17, or the pharmaceutically acceptable salt thereof, wherein the sense strand comprises any one of the sequences of the sense strands in Table 2.

**21.** The siRNA according to any one of claims 1-20, or the pharmaceutically acceptable salt thereof, wherein the sense strand comprises any one of the sequences of SEQ ID NOs: 255-277 and 541-543.

**22.** The siRNA according to any one of claims 1-21, or the pharmaceutically acceptable salt thereof, wherein the siRNA comprises any one of the siRNAs in Table 2.

**23.** The siRNA according to any one of claims 1-22, or the pharmaceutically acceptable salt thereof, wherein in the siRNA:

the antisense strand comprises SEQ ID NO: 380, and the sense strand comprises SEQ ID NO: 255;
the antisense strand comprises SEQ ID NO: 381, and the sense strand comprises SEQ ID NO: 256;
the antisense strand comprises SEQ ID NO: 382, and the sense strand comprises SEQ ID NO: 257;

the antisense strand comprises SEQ ID NO: 383, and the sense strand comprises SEQ ID NO: 258;
the antisense strand comprises SEQ ID NO: 384, and the sense strand comprises SEQ ID NO: 259;
the antisense strand comprises SEQ ID NO: 385, and the sense strand comprises SEQ ID NO: 260;
the antisense strand comprises SEQ ID NO: 386, and the sense strand comprises SEQ ID NO: 261;
the antisense strand comprises SEQ ID NO: 387, and the sense strand comprises SEQ ID NO: 262;
the antisense strand comprises SEQ ID NO: 388, and the sense strand comprises SEQ ID NO: 263;
the antisense strand comprises SEQ ID NO: 389, and the sense strand comprises SEQ ID NO: 264;
the antisense strand comprises SEQ ID NO: 390, and the sense strand comprises SEQ ID NO: 265;
the antisense strand comprises SEQ ID NO: 391, and the sense strand comprises SEQ ID NO: 266;
the antisense strand comprises SEQ ID NO: 392, and the sense strand comprises SEQ ID NO: 267;
the antisense strand comprises SEQ ID NO: 393, and the sense strand comprises SEQ ID NO: 268;
the antisense strand comprises SEQ ID NO: 394, and the sense strand comprises SEQ ID NO: 269;
the antisense strand comprises SEQ ID NO: 395, and the sense strand comprises SEQ ID NO: 270;
the antisense strand comprises SEQ ID NO: 396, and the sense strand comprises SEQ ID NO: 271;
the antisense strand comprises SEQ ID NO: 397, and the sense strand comprises SEQ ID NO: 272;
the antisense strand comprises SEQ ID NO: 398, and the sense strand comprises SEQ ID NO: 273;
the antisense strand comprises SEQ ID NO: 399, and the sense strand comprises SEQ ID NO: 274;
the antisense strand comprises SEQ ID NO: 400, and the sense strand comprises SEQ ID NO: 275;
the antisense strand comprises SEQ ID NO: 401, and the sense strand comprises SEQ ID NO: 276;
the antisense strand comprises SEQ ID NO: 402, and the sense strand comprises SEQ ID NO: 277;
the antisense strand comprises SEQ ID NO: 541, and the sense strand comprises SEQ ID NO: 544;
the antisense strand comprises SEQ ID NO: 542, and the sense strand comprises SEQ ID NO: 545; or
the antisense strand comprises SEQ ID NO: 543, and the sense strand comprises SEQ ID NO: 546.

**24.** The siRNA according to any one of claims 1-23, or the pharmaceutically acceptable salt thereof, wherein the sense strand and/or the antisense strand independently comprise one or more modified nucleotides.

**25.** The siRNA according to any one of claims 1-24, or the pharmaceutically acceptable salt thereof, wherein the modified nucleotides are 2'-modified nucleotides.

**26.** The siRNA according to any one of claims 1-25, or the pharmaceutically acceptable salt thereof, wherein the modified nucleotides comprise: 2'-O-methyl modified nucleotides, 2'-O-methoxyethyl modified nucleotides, 2'-fluoro modified nucleotides, 2'-deoxy modified nucleotides, 2'-O-alkenyl modified nucleotides, locked nucleotides, GNA, LNA, abasic nucleotides, deoxythymidine, inverted deoxythymidine, deoxyadenosine, inverted deoxyadenosine, 2'-amino-modified nucleotides, 2'-alkyl-modified nucleotides, morpholino nucleotides, nucleotides comprising non-natural bases, 5'-(E)-vinylphosphonate modified nucleotides, or a combination thereof.

**27.** The siRNA according to any one of claims 1-26, or the pharmaceutically acceptable salt thereof, wherein nucleotides thereof are all modified nucleotides.

**28.** The siRNA according to any one of claims 1-27, or the pharmaceutically acceptable salt thereof, wherein the modified nucleotides comprise 2'-O-methyl modified nucleotides, 2'-fluoro modified nucleotides, deoxythymidine, and 5'-(E)-vinylphosphonate modified nucleotides, or a combination thereof.

**29.** The siRNA according to any one of claims 1-28, or the pharmaceutically acceptable salt thereof, wherein the sense strand, the antisense strand, or both the sense strand and the antisense strand comprise one or more phosphorothioate internucleoside linkages or phosphorodithioate internucleoside linkages.

**30.** The siRNA according to claim 29, or the pharmaceutically acceptable salt thereof, wherein the antisense strand comprises two consecutive phosphorothioate internucleoside linkages between terminal nucleotides at the 3' end and between terminal nucleotides at the 5' end.

**31.** The siRNA according to claim 29 or 30, or the pharmaceutically acceptable salt thereof, wherein the sense strand comprises two consecutive phosphorothioate internucleoside linkages between terminal nucleotides at the 3' end and between terminal nucleotides at the 5' end.

**32.** The siRNA according to claim 29 or 30, or the pharmaceutically acceptable salt thereof, wherein the sense strand comprises two consecutive phosphorothioate internucleoside linkages between terminal nucleotides at the 5' end,

and the sense strand comprises one phosphorothioate internucleoside linkage between terminal nucleotides at the 3' end.

**33.** The siRNA according to claim 29 or 30, or the pharmaceutically acceptable salt thereof, wherein the sense strand comprises two consecutive phosphorothioate internucleoside linkages between terminal nucleotides at the 5' end, and the sense strand comprises one phosphorothioate internucleoside linkage between terminal nucleotides at the 3' end; and the antisense strand comprises two consecutive phosphorothioate internucleoside linkages between terminal nucleotides at the 3' end and between terminal nucleotides at the 5' end.

**34.** The siRNA according to any one of claims 1-33, or the pharmaceutically acceptable salt thereof, wherein:

1) the sense strand comprises 21 nucleotides, the antisense strand comprises 23 nucleotides, the duplex complementary region is 21 base pairs in length, nucleotides at positions 9, 10, and 11 from the 5' end of the sense strand are all 2'-fluoro modified nucleotides, and other nucleotides of the sense strand are all 2'-O-methyl modified nucleotides; nucleotides at positions 8, 10, 18, and 22 from the 3' end of the antisense strand are all 2'-fluoro modified nucleotides, and other nucleotides of the antisense strand are all 2'-O-methyl modified nucleotides; phosphorothioate linkages are used between nucleotides at positions 1 to 3 from the 5' end of the sense strand, between nucleotides at positions 1 to 3 from the 3' end of the sense strand, between nucleotides at positions 1 to 3 from the 5' end of the antisense strand, and between nucleotides at positions 1 to 3 from the 3' end of the antisense strand, and phosphodiester linkages are used between other nucleotides of the sense strand and between other nucleotides of the antisense strand;
2) the sense strand comprises 19 nucleotides, the antisense strand comprises 21 nucleotides, the duplex complementary region is 19 base pairs in length, nucleotides at positions 7, 8, and 9 from the 5' end of the sense strand are all 2'-fluoro modified nucleotides, and other nucleotides of the sense strand are all 2'-O-methyl modified nucleotides; positions 1 and 2 from the 3' end of the antisense strand are two dTs, nucleotides at positions 6, 8, 14, 16, and 20 from the 3' end of the antisense strand are all 2'-fluoro modified nucleotides, and other nucleotides of the antisense strand are all 2'-O-methyl modified nucleotides; phosphorothioate linkages are used between nucleotides at positions 1 to 3 from the 5' end of the sense strand, between nucleotides at positions 1 to 3 from the 3' end of the sense strand, between nucleotides at positions 1 to 3 from the 5' end of the antisense strand, and between nucleotides at positions 1 to 3 from the 3' end of the antisense strand, and phosphodiester linkages are used between other nucleotides of the sense strand and between other nucleotides of the antisense strand;
3) the sense strand comprises 19 nucleotides, the antisense strand comprises 21 nucleotides, the duplex complementary region is 19 base pairs in length, nucleotides at positions 7, 8, and 9 from the 5' end of the sense strand are all 2'-fluoro modified nucleotides, and other nucleotides of the sense strand are all 2'-O-methyl modified nucleotides; positions 1 and 2 from the 3' end of the antisense strand are two dTs, nucleotides at positions 4, 6, 8, 16, and 20 from the 3' end of the antisense strand are all 2'-fluoro modified nucleotides, and other nucleotides of the antisense strand are all 2'-O-methyl modified nucleotides; phosphorothioate linkages are used between nucleotides at positions 1 to 3 from the 5' end of the sense strand, between nucleotides at positions 1 to 3 from the 3' end of the sense strand, between nucleotides at positions 1 to 3 from the 5' end of the antisense strand, and between nucleotides at positions 1 to 3 from the 3' end of the antisense strand, and phosphodiester linkages are used between other nucleotides of the sense strand and between other nucleotides of the antisense strand;
4) the sense strand comprises 21 nucleotides, the antisense strand comprises 21 nucleotides, the duplex complementary region is 21 base pairs in length, nucleotides at positions 9, 10, and 11 from the 5' end of the sense strand are all 2'-fluoro modified nucleotides, and other nucleotides of the sense strand are all 2'-O-methyl modified nucleotides; nucleotides at positions 4, 6, 8, 10, 16, 18, and 20 starting from position 3 from the 3' end of the antisense strand are all 2'-fluoro modified nucleotides, and other nucleotides of the antisense strand are 2'-O-methyl modified nucleotides; phosphorothioate linkages are used between nucleotides at positions 1 to 3 from the 5' end of the sense strand, between nucleotides at positions 1 to 3 from the 3' end of the sense strand, between nucleotides at positions 1 to 3 from the 5' end of the antisense strand, and between nucleotides at positions 1 to 3 from the 3' end of the antisense strand, and phosphodiester linkages are used between other nucleotides of the sense strand and between other nucleotides of the antisense strand;
5) the sense strand comprises 21 nucleotides, the antisense strand comprises 21 nucleotides, the duplex complementary region is 21 base pairs in length, nucleotides at positions 9, 10, and 11 from the 5' end of the sense strand are all 2'-fluoro modified nucleotides, and other nucleotides of the sense strand are all 2'-O-methyl modified nucleotides; nucleotides at positions 4, 6, 8, 10, 16, 18, and 20 starting from position 3 from the 3' end of the antisense strand are all 2'-fluoro modified nucleotides, other nucleotides of the antisense strand are 2'-O-methyl modified nucleotides, and nucleotide at position 1 from the 5' end of the antisense strand is modified with

VP; phosphorothioate linkages are used between nucleotides at positions 1 to 3 from the 5' end of the sense strand, between nucleotides at positions 1 to 3 from the 3' end of the sense strand, between nucleotides at positions 1 to 3 from the 5' end of the antisense strand, and between nucleotides at positions 1 to 3 from the 3' end of the antisense strand, and phosphodiester linkages are used between other nucleotides of the sense strand and between other nucleotides of the antisense strand;

6) the sense strand comprises 19 nucleotides, the antisense strand comprises 21 nucleotides, the duplex complementary region is 19 base pairs in length, nucleotides at positions 7, 8, and 9 from the 5' end of the sense strand are all 2'-fluoro modified nucleotides, and other nucleotides of the sense strand are all 2'-O-methyl modified nucleotides; positions 1 and 2 from the 3' end of the antisense strand are two dTs, nucleotides at positions 6, 8, 14, 16, and 20 from the 3' end of the antisense strand are all 2'-fluoro modified nucleotides, other nucleotides of the antisense strand are all 2'-O-methyl modified nucleotides, and nucleotide at position 1 from the 5' end of the antisense strand is modified with VP; phosphorothioate linkages are used between nucleotides at positions 1 to 3 from the 5' end of the sense strand, between nucleotides at positions 1 to 3 from the 3' end of the sense strand, between nucleotides at positions 1 to 3 from the 5' end of the antisense strand, and between nucleotides at positions 1 to 3 from the 3' end of the antisense strand, and phosphodiester linkages are used between other nucleotides of the sense strand and between other nucleotides of the antisense strand;

7) the sense strand comprises 19 nucleotides, the antisense strand comprises 21 nucleotides, the duplex complementary region is 19 base pairs in length, nucleotides at positions 7, 8, and 9 from the 5' end of the sense strand are all 2'-fluoro modified nucleotides, and other nucleotides of the sense strand are all 2'-O-methyl modified nucleotides; positions 1 and 2 from the 3' end of the antisense strand are two dTs, nucleotides at positions 6, 8, 14, 16, and 20 from the 3' end of the antisense strand are all 2'-fluoro modified nucleotides, and other nucleotides of the antisense strand are all 2'-O-methyl modified nucleotides; phosphorothioate linkages are used between nucleotides at positions 1 to 3 from the 5' end of the sense strand, between nucleotides at positions 1 to 2 from the 3' end of the sense strand, between nucleotides at positions 1 to 3 from the 5' end of the antisense strand, and between nucleotides at positions 1 to 3 from the 3' end of the antisense strand, and phosphodiester linkages are used between other nucleotides of the sense strand and between other nucleotides of the antisense strand; or

8) the sense strand comprises 19 nucleotides, the antisense strand comprises 21 nucleotides, the duplex complementary region is 19 base pairs in length, nucleotides at positions 7, 8, and 9 from the 5' end of the sense strand are all 2'-fluoro modified nucleotides, and other nucleotides of the sense strand are all 2'-O-methyl modified nucleotides; positions 1 and 2 from the 3' end of the antisense strand are two dTs, nucleotides at positions 6, 8, 14, 16, and 20 from the 3' end of the antisense strand are all 2'-fluoro modified nucleotides, other nucleotides of the antisense strand are all 2'-O-methyl modified nucleotides, and nucleotide at position 1 from the 5' end of the antisense strand is modified with VP; phosphorothioate linkages are used between nucleotides at positions 1 to 3 from the 5' end of the sense strand, between nucleotides at positions 1 to 2 from the 3' end of the sense strand, between nucleotides at positions 1 to 3 from the 5' end of the antisense strand, and between nucleotides at positions 1 to 3 from the 3' end of the antisense strand, and phosphodiester linkages are used between other nucleotides of the sense strand and between other nucleotides of the antisense strand.

**35.** The siRNA according to any one of claims 1-34, or the pharmaceutically acceptable salt thereof, wherein the antisense strand comprises any one of the antisense strands in Table 3.

**36.** The siRNA according to any one of claims 1-35, or the pharmaceutically acceptable salt thereof, wherein the antisense strand comprises any one of the antisense strands of SEQ ID NOs: 505, 507, 509, 511, 513, 515, 517, 519, 521, 523, 525, and 527.

**37.** The siRNA according to any one of claims 1-36, or the pharmaceutically acceptable salt thereof, wherein the sense strand comprises any one of the sense strands in Table 3.

**38.** The siRNA according to any one of claims 1-37, or the pharmaceutically acceptable salt thereof, wherein the sense strand comprises any one of the sense strands of SEQ ID NOs: 506, 508, 510, 512, 514, 516, 518, 520, 522, 524, 526, and 528.

**39.** The siRNA according to any one of claims 1-38, or the pharmaceutically acceptable salt thereof, wherein the siRNA comprises any one of the siRNAs in Table 3.

**40.** The siRNA according to any one of claims 1-39, or the pharmaceutically acceptable salt thereof, wherein in the siRNA:

the antisense strand comprises SEQ ID NO: 505, and the sense strand comprises SEQ ID NO: 506;
the antisense strand comprises SEQ ID NO: 507, and the sense strand comprises SEQ ID NO: 508;
the antisense strand comprises SEQ ID NO: 509, and the sense strand comprises SEQ ID NO: 510;
the antisense strand comprises SEQ ID NO: 511, and the sense strand comprises SEQ ID NO: 512;
the antisense strand comprises SEQ ID NO: 513, and the sense strand comprises SEQ ID NO: 514;
the antisense strand comprises SEQ ID NO: 515, and the sense strand comprises SEQ ID NO: 516;
the antisense strand comprises SEQ ID NO: 517, and the sense strand comprises SEQ ID NO: 518;
the antisense strand comprises SEQ ID NO: 519, and the sense strand comprises SEQ ID NO: 520;
the antisense strand comprises SEQ ID NO: 521, and the sense strand comprises SEQ ID NO: 522;
the antisense strand comprises SEQ ID NO: 523, and the sense strand comprises SEQ ID NO: 524;
the antisense strand comprises SEQ ID NO: 525, and the sense strand comprises SEQ ID NO: 526; or
the antisense strand comprises SEQ ID NO: 527, and the sense strand comprises SEQ ID NO: 528.

**41.** The siRNA according to any one of claims 1-40, or the pharmaceutically acceptable salt thereof, wherein the siRNA further comprises a targeting group.

**42.** The siRNA according to claim 41, or the pharmaceutically acceptable salt thereof, wherein the targeting group comprises an asialoglycoprotein receptor ligand.

**43.** The siRNA according to claim 42, or the pharmaceutically acceptable salt thereof, wherein the asialoglycoprotein receptor ligand comprises a galactose cluster.

**44.** The siRNA according to claim 43, or the pharmaceutically acceptable salt thereof, wherein the galactose cluster is a monovalent, divalent, trivalent, or tetravalent galactose derivative.

**45.** The siRNA according to claim 44, or the pharmaceutically acceptable salt thereof, wherein the galactose derivative is N-acetylgalactosamine (GalNAc).

**46.** The siRNA according to claim 45, or the pharmaceutically acceptable salt thereof, wherein the galactose cluster is a trivalent N-acetylgalactosamine.

**47.** The siRNA according to any one of claims 1-46, or the pharmaceutically acceptable salt thereof, wherein the galactose cluster is linked to a branching center via a linker, and the branching center is conjugated to a nucleotide of the siRNA via a linker.

**48.** The siRNA according to any one of claims 1-47, or the pharmaceutically acceptable salt thereof, wherein the targeting group is conjugated to the 5' end of the sense strand.

**49.** The siRNA according to any one of claims 1-47, or the pharmaceutically acceptable salt thereof, wherein the targeting group is conjugated to the 3' end of the sense strand.

**50.** The siRNA according to any one of claims 1-47, or the pharmaceutically acceptable salt thereof, wherein a fragment comprising the targeting group as follows is conjugated to the 5' end or the 3' end of the sense strand:

or,

51. The siRNA according to claim 50, or the pharmaceutically acceptable salt thereof, wherein the fragment comprising the targeting group is conjugated to the 5' end of the sense strand.

52. The siRNA according to claim 51, or the pharmaceutically acceptable salt thereof, wherein the fragment comprising the targeting group is conjugated to the 3' end of the sense strand.

53. The siRNA according to any one of claims 1-52, or the pharmaceutically acceptable salt thereof, wherein the siRNA comprises any one of the siRNAs in Table 4.

54. The siRNA according to claim 53, or the pharmaceutically acceptable salt thereof, wherein the sense strand is any one of the sense strands in Table 4.

55. The siRNA according to claim 53, or the pharmaceutically acceptable salt thereof, wherein the antisense strand is any one of the antisense strands in Table 4.

56. A pharmaceutical composition, comprising the siRNA according to any one of claims 1-55 or the pharmaceutically acceptable salt thereof, and a pharmaceutically acceptable salt.

57. A method of reducing the expression level of LPA in a subject, wherein the method comprises administering to the subject the siRNA according to any one of claims 1-55 or the pharmaceutically acceptable salt thereof, or the pharmaceutical composition according to claim 56.

58. Use of the siRNA according to any one of claims 1-55 or the pharmaceutically acceptable salt thereof, or the pharmaceutical composition according to claim 56, in the manufacture of a medicament for preventing or treating a disease associated with LPA expression.

59. The use according to claim 58, wherein the disease associated with LPA expression is a cardiovascular and cerebrovascular disease.

60. The use according to claim 59, wherein the cardiovascular and cerebrovascular disease comprises stroke, atherosclerosis, thrombosis, coronary heart disease, or aortic stenosis, and any other disease or pathology associated with elevated levels of Lp(a) particles.

61. A method of preventing or treating a disease associated with LPA expression, wherein the method comprises administering to a subject the siRNA according to any one of claims 1-55 or the pharmaceutically acceptable salt thereof, or the pharmaceutical composition according to claim 56.

62. The method according to claim 61, wherein the disease associated with LPA expression is a cardiovascular and cerebrovascular disease.

**63.** The method according to claim 62, wherein the cardiovascular and cerebrovascular disease comprises stroke, atherosclerosis, thrombosis, coronary heart disease, or aortic stenosis, and any other disease or pathology associated with elevated levels of Lp(a) particles.

**64.** The siRNA according to any one of claims 1-55 or the pharmaceutically acceptable salt thereof, or the pharmaceutical composition according to claim 56, for use in preventing or treating a disease associated with LPA expression.

**65.** The use according to claim 64, wherein the disease associated with LPA expression is a cardiovascular and cerebrovascular disease.

**66.** The use according to claim 65, wherein the cardiovascular and cerebrovascular disease comprises stroke, atherosclerosis, thrombosis, coronary heart disease, or aortic stenosis, and any other disease or pathology associated with elevated levels of Lp(a) particles.

To cycle n+1

DMTO

Base

$Base_{n-1}$

R'

CN

1, Detritylation

Cleavage&Deprotection

HO

2, coupling

3, capping

4, oxidation

phosphoramidites monoers

FIG. 1

1000
100
10
1
Lp(a) Level (%)
0
7
14
21
28
35
42
49
56
Days
PBS
GalNAC-SLN360 1mg/kg
JAB-190024-1 3mg/kg
JAB-190024-1 1mg/kg
JAB-190024-3 3mg/kg
JAB-190024-3 1mg/kg
JAB-190024-5 3mg/kg
JAB-190024-5 1mg/kg

FIG. 2

1000
100
10
1
Lp(a) Level (%)
0
7
14
21
28
35
42
49
56
63
70
77
84
Days
PBS
NAG25-AMG890 1mg/kg
GalNAC-SLN360 1mg/kg
JAB-190024-1 0.3mg/kg
JAB-190024-1 1mg/kg
JAB-190024-2 0.3mg/kg
JAB-190024-2 1mg/kg
JAB-190024-4 1mg/kg
JAB-190024-6 1mg/kg

FIG. 3

120
100
80
60
40
20
0
Lp(a) Level (%)
PRE
7
14
21
Days
JAB-190024-1, 1 mg/kg
JAB-190024-2, 1 mg/kg
JAB-190024-6, 1 mg/kg

FIG. 4

**INTERNATIONAL SEARCH REPORT**

International application No. **PCT/CN2024/131910**

**A. CLASSIFICATION OF SUBJECT MATTER**

C12N15/113(2010.01)i; A61K31/713(2006.01)i; A61P9/00(2006.01)i; A61K48/00(2006.01)i

According to International Patent Classification (IPC) or to both national classification and IPC

**B. FIELDS SEARCHED**

Minimum documentation searched (classification system followed by classification symbols)

IPC: C12N; A61K; A61P

Documentation searched other than minimum documentation to the extent that such documents are included in the fields searched

Electronic data base consulted during the international search (name of data base and, where practicable, search terms used)

CJFD, CNTXT, ENTXTC, DWPI, VEN, CNKI, BING, NCBI, EBI, STN, 百度学术, Baidu Scholar, 中国专利生物序列检索系统, China Patent Biological Sequence Search System: LPA, 脂蛋白, apo(a), lipoprotein a, lp(a), siRNA, shRNA, dsRNA, 抑制剂, 敲低, 敲减, 删除, dT, 悬垂, 修饰, SLN360, galNAC, SEQ ID NOs.1-12, 128-139, 255-266, 380-391 and 505-540

**C. DOCUMENTS CONSIDERED TO BE RELEVANT**

| Category* | Citation of document, with indication, where appropriate, of the relevant passages | Relevant to claim No. |
|---|---|---|
| PX | CN 117327698 A (BEIJING FOYOU PHARMACEUTICAL CO., LTD.) 02 January 2024 (2024-01-02)<br>claims 1-45, and sequence listing | 1-66 |
| PX | WO 2024088190 A1 (KYLONOVA (XIAMEN) BIOPHARMA CO., LTD.) 02 May 2024 (2024-05-02)<br>claims 1-13, and sequence listing | 1-66 |
| PX | WO 2024035898 A2 (SIRIUS THERAPEUTICS, INC.) 15 February 2024 (2024-02-15)<br>claims 1-35, and sequence listing | 1-66 |
| X | WO 2023041079 A1 (GENOVAL THERAPEUTICS CO., LTD.) 23 March 2023 (2023-03-23)<br>claims 1-22, and sequence listing | 1-66 |
| X | WO 2023041508 A2 (ARGONAUTE RNA LIMITED) 23 March 2023 (2023-03-23)<br>claims 1-32, and sequence listing | 1-66 |
| X | CN 111465694 A (SILENCE THERAPEUTICS GMBH) 28 July 2020 (2020-07-28)<br>claims 1-22, and sequence listing | 1-66 |

☑ Further documents are listed in the continuation of Box C. ☑ See patent family annex.

* Special categories of cited documents:

"A" document defining the general state of the art which is not considered to be of particular relevance

"D" document cited by the applicant in the international application

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **27 February 2025** | **06 March 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/131910**

| C. DOCUMENTS CONSIDERED TO BE RELEVANT | | |
|---|---|---|
| Category* | Citation of document, with indication, where appropriate, of the relevant passages | Relevant to claim No. |
| X | CN 108368506 A (ARROWHEAD PHARMACEUTICALS, INC.) 03 August 2018 (2018-08-03)<br>claims 1-31, and sequence listing | 1-66 |
| X | WO 2021119034 A1 (AMGEN INC.) 17 June 2021 (2021-06-17)<br>claims 1-63 | 1-66 |
| X | CN 116490195 A (SANOFI-AVENTIS) 25 July 2023 (2023-07-25)<br>claims 1-39 | 1-66 |
| A | O'DONOGHUE, M.L. et al. "Small Interfering RNA to Reduce Lipoprotein(a) in Cardiovascular Disease"<br>*The New England Journal of Medicine,*<br>Vol. 387, No. (20), 17 November 2022 (2022-11-17), 1855-1864<br>abstract, page 1862, right-hand column, last paragraph to page 1863, right-hand column, last paragraph | 1-66 |
| A | RIDER, D. et al. "Preclinical Toxicological Assessment of a Novel siRNA, SLN360, Targeting Elevated Lipoprotein(a) in Cardiovascular Disease"<br>*Toxicological Sciences,* Vol. 189, No. (2), 23 June 2022 (2022-06-23), 237-249<br>abstract, page 240, left-hand column, last paragraph to right-hand column, last paragraph | 1-66 |

Form PCT/ISA/210 (second sheet) (July 2022)

**INTERNATIONAL SEARCH REPORT**

International application No.
**PCT/CN2024/131910**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/131910**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **57, 61-63**
because they relate to subject matter not required to be searched by this Authority, namely:

Claims 57 and 61-63 relate to a method for treating diseases, which falls within the cases set out in PCT Rule 39.1(iv) for which no search is required by the International Searching Authority.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/131910**

| Patent document cited in search report | Publication date (day/month/year) | Patent family member(s) | Publication date (day/month/year) |
|---|---|---|---|
| CN 117327698 A | 02 January 2024 | None | |
| WO 2024088190 A1 | 02 May 2024 | TW 202426007 A | 01 July 2024 |
| | | TWI 863646 B | 21 November 2024 |
| WO 2024035898 A2 | 15 February 2024 | WO 2024035898 A3 | 28 March 2024 |
| | | TW 202417624 A | 01 May 2024 |
| WO 2023041079 A1 | 23 March 2023 | CA 3232191 A1 | 23 March 2023 |
| | | AU 2022348141 A1 | 04 April 2024 |
| | | EP 4403633 A1 | 24 July 2024 |
| | | KR 20240056619 A | 30 April 2024 |
| | | US 2024271140 A1 | 15 August 2024 |
| | | WO 2023041079 A9 | 01 June 2023 |
| | | JP 2024534508 A | 20 September 2024 |
| WO 2023041508 A2 | 23 March 2023 | CA 3229020 A1 | 23 March 2023 |
| | | WO 2023041508 A3 | 08 June 2023 |
| | | JP 2024531728 A | 29 August 2024 |
| | | EP 4402263 A2 | 24 July 2024 |
| CN 111465694 A | 28 July 2020 | HUE 061265 T2 | 28 June 2023 |
| | | FI 3710586 T3 | 02 March 2023 |
| | | HRP 20230127 T1 | 31 March 2023 |
| | | KR 20200088392 A | 22 July 2020 |
| | | KR 102553382 B1 | 07 July 2023 |
| | | ZA 202003371 B | 30 August 2023 |
| | | MX 2020004897 A | 05 October 2020 |
| | | AU 2018363840 A1 | 16 April 2020 |
| | | AU 2018363840 B2 | 29 September 2022 |
| | | SI 3710586 T1 | 31 March 2023 |
| | | BR 112020009152 A2 | 27 October 2020 |
| | | EP 4219716 A2 | 02 August 2023 |
| | | EP 4219716 A3 | 27 March 2024 |
| | | WO 2019092283 A1 | 16 May 2019 |
| | | PL 3710586 T3 | 20 March 2023 |
| | | US 2022290144 A1 | 15 September 2022 |
| | | US 12054717 B2 | 06 August 2024 |
| | | DK 3710586 T3 | 23 January 2023 |
| | | US 2021123048 A1 | 29 April 2021 |
| | | US 11319537 B2 | 03 May 2022 |
| | | SG 11202003230 UA | 28 May 2020 |
| | | LT 3710586 T | 27 February 2023 |
| | | CA 3081905 A1 | 16 May 2019 |
| | | CA 3081905 C | 03 October 2023 |
| | | EP 3710586 A1 | 23 September 2020 |
| | | EP 3710586 B1 | 23 November 2022 |
| | | JP 2021502120 A | 28 January 2021 |
| | | JP 7245254 B2 | 23 March 2023 |
| | | ES 2938193 T3 | 05 April 2023 |
| | | IL 274503 A | 30 June 2020 |
| | | IL 274503 B1 | 01 June 2024 |
| | | IL 274503 B2 | 01 October 2024 |
| | | US 2024327843 A1 | 03 October 2024 |

Form PCT/ISA/210 (patent family annex) (July 2022)

**INTERNATIONAL SEARCH REPORT**
**Information on patent family members**

International application No.
**PCT/CN2024/131910**

| Patent document cited in search report | Publication date (day/month/year) | Patent family member(s) | Publication date (day/month/year) |
|---|---|---|---|
| | | RS 63955 B1 | 28 February 2023 |
| CN 108368506 A | 03 August 2018 | MX 2018003833 A | 18 June 2018 |
| | | HK 1259063 A1 | 22 November 2019 |
| | | TN 2018000094 A1 | 08 July 2019 |
| | | MY 195796 A | 21 February 2023 |
| | | HUE 055942 T2 | 28 January 2022 |
| | | CY 1125263 T1 | 24 March 2023 |
| | | UY 36926 A | 28 April 2017 |
| | | SMT 202100622 T1 | 10 January 2022 |
| | | ZA 202106265 B | 30 August 2023 |
| | | TW 202332769 A | 16 August 2023 |
| | | TWI 836693 B | 21 March 2024 |
| | | IL 300438 A | 01 April 2023 |
| | | CA 3000397 A1 | 06 April 2017 |
| | | PE 20181139 A1 | 17 July 2018 |
| | | EA 201890864 A1 | 28 September 2018 |
| | | EA 038478 B1 | 03 September 2021 |
| | | US 2020263179 A1 | 20 August 2020 |
| | | IL 258333 A | 31 May 2018 |
| | | IL 258333 B | 01 March 2022 |
| | | HRP 20211410 T1 | 24 December 2021 |
| | | CR 20180231 A | 31 May 2018 |
| | | WO 2017059223 A2 | 06 April 2017 |
| | | WO 2017059223 A3 | 11 May 2017 |
| | | WO 2017059223 A9 | 17 August 2017 |
| | | ES 2896298 T3 | 24 February 2022 |
| | | SG 10202008530 TA | 29 October 2020 |
| | | EP 3356529 A2 | 08 August 2018 |
| | | EP 3356529 A4 | 04 September 2019 |
| | | EP 3356529 B1 | 25 August 2021 |
| | | RS 62523 B1 | 30 November 2021 |
| | | US 2018195070 A1 | 12 July 2018 |
| | | US 10662427 B2 | 26 May 2020 |
| | | EP 4029941 A1 | 20 July 2022 |
| | | US 2017096665 A1 | 06 April 2017 |
| | | US 9932586 B2 | 03 April 2018 |
| | | IL 290566 A | 01 April 2022 |
| | | IL 290566 B1 | 01 March 2023 |
| | | IL 290566 B2 | 01 July 2023 |
| | | TW 201726918 A | 01 August 2017 |
| | | TWI 784934 B | 01 December 2022 |
| | | LT 3356529 T | 27 December 2021 |
| | | KR 20180052703 A | 18 May 2018 |
| | | KR 102728481 B1 | 12 November 2024 |
| | | KR 20240162596 A | 15 November 2024 |
| | | MA 43347 A | 08 August 2018 |
| | | MA 43347 B1 | 30 November 2021 |
| | | BR 112018006489 A2 | 09 October 2018 |
| | | UA 121998 C2 | 25 August 2020 |
| | | JP 2021087459 A | 10 June 2021 |

Form PCT/ISA/210 (patent family annex) (July 2022)

**INTERNATIONAL SEARCH REPORT**
**Information on patent family members**

International application No.
**PCT/CN2024/131910**

| Patent document cited in search report | Publication date (day/month/year) | Patent family member(s) | Publication date (day/month/year) |
|---|---|---|---|
| | | JP 7116212 B2 | 09 August 2022 |
| | | CO 2018003678 A2 | 30 November 2018 |
| | | JP 2018529732 A | 11 October 2018 |
| | | JP 6991966 B2 | 03 February 2022 |
| | | MX 2022013010 A | 09 November 2022 |
| | | PH 12018500713 A1 | 15 October 2018 |
| | | JP 2024009262 A | 19 January 2024 |
| | | AU 2022283623 A1 | 02 February 2023 |
| | | SI 3356529 T1 | 31 January 2022 |
| | | PL 3356529 T3 | 20 December 2021 |
| | | DK 3356529 T3 | 08 November 2021 |
| | | JP 2022113835 A | 04 August 2022 |
| | | JP 7442574 B2 | 04 March 2024 |
| | | JOP 20210043 A1 | 16 June 2017 |
| | | AU 2016331084 A1 | 19 April 2018 |
| | | AU 2016331084 B2 | 08 September 2022 |
| | | PT 3356529 T | 04 November 2021 |
| | | NZ 741086 A | 24 November 2023 |
| | | JOP 20160211 B1 | 17 August 2021 |
| | | CL 2018000803 A1 | 31 August 2018 |
| WO 2021119034 A1 | 17 June 2021 | AU 2020399636 A1 | 02 June 2022 |
| | | MX 2022007005 A | 25 August 2022 |
| | | EP 4073252 A1 | 19 October 2022 |
| | | JP 2023504744 A | 06 February 2023 |
| | | US 2023078200 A1 | 16 March 2023 |
| | | CA 3163322 A1 | 17 June 2021 |
| CN 116490195 A | 25 July 2023 | WO 2022079221 A1 | 21 April 2022 |
| | | US 2024035029 A1 | 01 February 2024 |
| | | EP 4229201 A1 | 23 August 2023 |
| | | JP 2023545502 A | 30 October 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 117327698 A
- CN 116801886 A
- US 9932586 B
- US 11499153 B